# EUROPEAN PATENT SPECIFICATION

(11) **EP 1 263 973 B1**
(45) Date of publication and mention of the grant of the patent: **09.08.2006**
(21) Application number: 01914857.6
(22) Date of filing: 07.03.2001
(51) Int. Cl.: C12N 15/66, C12Q 1/68

(54) **DNA JOINING METHOD**
VERFAHREN ZUR VERBINDUNG VON DNA
PROCEDE D'ASSEMBLAGE D'ADN

(30) Priority: 07.03.2000 US 187401 P; 01.09.2000 CA 2317865; 25.01.2001 US 263771 P
(43) Date of publication of application: 11.12.2002
(73) Proprietor: UNIVERSITY OF GUELPH, Guelph Ontario N1G 5G3 (CA)
(72) Inventor: EVANS, David, H., Carlisle, Ontario L0R 1H2 (CA); WILLER, David, O., Guelph, Ontario N1G 4G7 (CA); YAO, Xiao-Dan, Guelph, Ontario N1G 2V7 (CA)
(74) Representative: Chapman, Paul Gilmour
(86) International application number: PCT/CA2001/000283
(87) International publication number: WO 2001/066775

(56) References cited:
- WO-A-98/38297
- US-A- 5 580 759
- US-A- 5 773 257
- KUIJPER J L ET AL: "FUNCTIONAL CLONING VECTORS FOR USE IN DIRECTIONAL CDNA CLONING USING COHESIVE ENDS PRODUCED WITH T4 DNA POLYMERASE" GENE (AMSTERDAM), vol. 112, no. 2, 1992, pages 147-155, XP001015913 ISSN: 0378-1119 cited in the application
- ASLANIDIS C ET AL: "LIGATION-INDEPENDENT CLONING OF PCR PRODUCTS (LIC-PCR)" NUCLEIC ACIDS RESEARCH, OXFORD UNIVERSITY PRESS, SURREY, GB, vol. 18, no. 20, 25 October 1990 (1990-10-25), pages 6069-6074, XP000159869 ISSN: 0305-1048 cited in the application
- HAUN R S ET AL: "LIGATION-INDEPENDENT CLONING OF GLUTATHIONE S-TRANSFERASE FUSION GENES FOR EXPRESSION IN ESCHERICHIA-COLI" GENE (AMSTERDAM), vol. 112, no. 1, 1992, pages 37-43, XP001015912 ISSN: 0378-1119
- TSENG HUNG: "DNA cloning without restriction enzyme and ligase." BIOTECHNIQUES, vol. 27, no. 6, December 1999 (1999-12), pages 1240-1244, XP001015911 ISSN: 0736-6205
- FIORENTINI PAOLA ET AL: "Exonuclease I of Saccharomyces cerevisiae functions in mitotic recombination in vivo and in vitro." MOLECULAR AND CELLULAR BIOLOGY, vol. 17, no. 5, 1997, pages 2764-2773, XP002176159 ISSN: 0270-7306
- SHIRAISHI H ET AL: "A RAPID AND EFFICIENT METHOD FOR TARGETED RANDOM MUTAGENESIS" GENE (AMSTERDAM), vol. 64, no. 2, 1988, pages 313-320, XP001016174 ISSN: 0378-1119
- WILLER DAVID O ET AL: "Vaccinia virus DNA polymerase promotes DNA pairing and strand-transfer reactions." VIROLOGY, vol. 257, no. 2, 10 May 1999 (1999-05-10), pages 511-523, XP001023042 ISSN: 0042-6822 cited in the application
- WILLER DAVID O ET AL: "In vitro concatemer formation catalyzed by vaccinia virus DNA polymerase." VIROLOGY, vol. 278, no. 2, 20 December 2000 (2000-12-20), pages 562-569, XP001023043 ISSN: 0042-6822

## Description

### FIELD OF THE INVENTION

The present invention is in the field of recombinant DNA technology. Specifically, the present invention relates to a method of directionally joining one or more DNA molecules using a DNA polymerase, and the application of this method to the cloning of PCR products.

### BACKGROUND OF THE INVENTION

The Polymerase Chain Reaction (PCR) (Mullis and Faloona 1987; Saiki, Gelfand et al. 1988) is an integral technique in scientific research. Cloning of PCR products is often an obligate step towards reaching a research objective. PCR-cloning presents numerous challenges and various techniques have been devised over the years to minimize its limitations. Cloning of PCR products generally fall into one of the following methodologies; i) traditional PCR-cloning using restriction enzymes and ligase, ii) T-vector or TA-cloning, iii) uracil DNA glycosylase (UDG)-based cloning iv) PCR-based techniques, v) in vivo recombinase methods, and vi) exonuclease-mediated cloning. Each of these methodologies is described below.

### i) Traditional PCR-cloning

Cloning of PCR-amplified DNA was traditionally facilitated by incorporating restriction endonuclease (RE) sites into the PCR primers, allowing for subsequent digestion of the PCR product with the appropriate enzyme followed by insertion into a compatible vector (Sharf et al., 1990). One problem often encountered with this method is that REs are notoriously poor cutters when their recognition sequences are close to the ends of the DNA substrate (Kaufman and Evans 1990). A second complication is that sequences within the PCR-amplified region can be lost if a second restriction site for the same RE is unknowingly present. This necessitates that the complete sequence of the PCR product be known prior to choosing which RE to use. Once the vector and DNA insert have been digested with the same RE, the two DNA molecules can be covalently joined by DNA ligase in a reaction typically taking 4-12hr. It should be noted that during any ligation reaction, it is critical that the vector and insert are present in appropriate ratios, which is often difficult to determine. The reaction products are then used to transform competent *E*. *coli* cells. A subtle variation on this theme called "ligation by overlap extension" has been devised which does not require any subsequent ligation reaction, but does require two additional primers, and the entire vector sequence itself must be amplified (Shuldiner, Tanner et al. 1991).

Blunt-end cloning of PCR fragments has also been used extensively, although this technique is relatively inefficient because of the problems encountered by DNA ligase when joining together two blunt-ended DNA molecules. This technique is also complicated by the fact that *Taq* polymerase (the prototypical PCR amplifying enzyme) has a propensity for adding 3' terminal adenine residues through its terminal transferase activity (Clark 1988; Mole, Iggo et al. 1989). Approximately 50% of all PCR products generated using *Taq* polymerase contain these 3'extensions (Clark, 1988). One way around this problem is to "polish" or remove these added adenine residues with Klenow (Hemsley, Arnheim et al. 1989) or T4 DNA polymerase (Costa and Weiner 1994), adding an additional step to the protocol.

Recent discoveries of different thermostable polymerases including *Pfu* polymerase from *Pyrococcus furiosis* and Vent_{R} polymerase from the archaebacteria *Thermococcus litoralis* do not produce 3' adenine residue extensions due to inherent 3'-5' exonuclease activities. In addition to the problems produced by the amplifying polymerase, this technique does not allow for directional cloning, meaning that the orientation of insert in the recombinant DNA cannot be predetermined.

### ii) T-Vector and TA-cloning

The terminal transferase activity of *Taq* polymerase has been exploited by many researchers in a technique now commonly known as TA-cloning. The chosen vector is digested with the appropriate RE so as to yield ends with protruding thymidine residues, the natural complement to the 3'-overhanging adenosine residues found on the PCR-amplified DNA. The most significant drawback of this technique is that vectors must be specifically engineered to produce compatible ends. The only simple way of accomplishing this goal is to restrict the vector to produce blunt ends, and then treat it with *Taq* polymerase in the presence of only dTTP's. Numerous companies have developed kits based on this technique, including pCR-Script™ SK(S) kit from Stratagene, pGEM®-T from Promega, the SureClone™ ligation kit from Pharmacia and the pT7Blue T-vector kit from Novagen. The main limitation to these methods is that a vector supplied by the manufacturer must be used and a second subcloning step is often necessary to move the cloned DNA fragment into a vector of choice. An inexpensive alternative to buying these kits is to use a T-vector pGEM®-5fZ(+) which is available for little or no cost from the American Type Culture Collection (ATCC). This vector when digested with *Xcm*I provides the T-overhangs used for TA-cloning (Kovalic, Kwak et al. 1991; Mead, Pey et al. 1991). Numerous other T-vectors have been developed independently (Cha. Bishai et al. 1993; Ichihara and Kurosawa 1993) which, after appropriate RE digestion, yield appropriate ends. Such vectors, however, required extensive manipulations to create. Some other potential problems with these kits have been reported recently (Hengen 1995). High backgrounds were observed for the pCR-Script vector when tested alone, PCRII contains a pBR322 origin of replication and thus replicate to low copy numbers, and repeated freeze-thaw cycles at -20°C can lead to instability and loss of the T-tails. All the T-vector techniques suffer from the drawback that they are non-directional and require a ligation step.

Invitrogen have improved upon traditional TA-cloning by bypassing the need for a ligation step. This method, called TOPO TA-cloning, takes advantage of a reaction catalyzed by a vaccinia virus enzyme called topoisomerase I (Shuman, 1994 and US 5,766,891). Topoisomerase can bind to double-stranded DNA and cleave the phosphodiester backbone in one of the duplex strands. The enzyme is sequence specific, cleaving primarily at the recognition sequence 5'-(C/T)CCTT↓-3' (Shuman and Prescott 1990; Shuman 1991). The enzyme is capable of re-ligating the original strand back together, or ligating two heterologous DNAs in the formation of a recombinant species (Shuman 1992; Shuman 1992). The reaction is very efficient requiring only a 5 minute benchtop incubation. The methodology also has advantages which obviates the need for ligase, does not require knowledge of the entire insert sequence and no additional nucleotides need be added to PCR primers. However, only specific plasmids engineered to contain the TOPO recognition sequence can be used. These vectors are produced by restricting the vector followed by adding specific linkers or adaptors, which is not a trivial task. Another limitation of this technique, is that the TOPO recognition sequence must be located within 10 bp from the 3'-ends of the vector, and furthermore, the insert must have a 5-OH group. The issue has been raised that internal recognition sequences within the amplified DNA may result in complications, however these sites are simply religated and do not impose any restrictions on this technique (Shuman 1994; Stivers, Shuman et al. 1994). Under general use, the Invitrogen kit provided another potential problem (unpublished results). The traditional method for screening clones, called blue-white selection, does not produce definitive results with the Invitrogen kit. Therefore, it is necessary to assay both white and light-blue colonies to ensure the correct construct is obtained.

### iii) Uracil DNA-glycosylase (UDG)-based cloning

Rashtchian et al., (1992) developed a ligase independent PCR cloning method using uracil DNA glycosylase (UDG), an enzyme whose normal cellular role is a DNA repair enzyme. The technique requires a 12-bp addition (CUACUACUACUA) to the 5'end of the PCR primers. The glycosylase selectively removes dUMP residues at the ends of the PCR products which disrupts proper base-pairing leading to single-strand 3'-overhangs (Duncan and Chambers 1984; Longo, Berninger et al. 1990). These 3'-overhangs can anneal to appropriately prepared single-strand ends of a vector. Uracil glycosylase is not active with thymine residues, the DNA counterpart of uracil residues (Duncan and Chambers, 1984), and is capable of removing dUMP residues even near the extreme ends (Varshney and van de Sande 1991). This methodology requires that the vector contain the appropriate complementary sequences, and is not amenable to use with proofreading polymerases such as *Pfu* or Vent_{R} polymerases (Sakaguchi, Sedlak et al. 1996). The researchers must therefore use *Taq* polymerase which has a significantly increased error frequency. UDG-based cloning has been commercialized by Life Technologies with their Clone Amp^{R} pUC system.

A variation on UDG-cloning takes advantage of the abasic sites (AP) produced by UDG-cleavage at dUMP residues. These AP sites are substrates for AP endonucleases such as T4 endonuclease V or human AP endonuclease I. Treatment with either of these repair enzymes yields a 5-P which is suitable for subsequent ligation into the appropriate vector. One drawback of this method is the requirement for a modified base (deoxyuridine) in the primer, and success relies on two enzymes in addition to ligase treatment. A second more obscure variation of UDG-cloning involves the use of a non-base residue called 1,3-propanediol in a predetermined position within the PCR primer, which can yield compatible 5'-ends for cloning, however, this method is much less efficient than other ligase-independent cloning methods (Kaluz and Flint 1994).

### iv) PCR-Directed Cloning

PCR-specific cloning methods arc often one-step procedures in which the recombinant DNA is produced during the amplification procedure itself. There are many variations on this theme, in which some are ligase-dependent and others are not. These methods are primarily used to produce site-specific mutations in cloned genes. A brief description of the current techniques follows.

### Ligase-Dependent Methods

a) Stratagene have commercialized a technique for the cloning of blunt-end PCR fragments (Weiner 1993), originally described by Liu and Schwartz (1992). Their methodology requires phosphorylating the 5' end of the PCR primers. The recipient vector is linearized and treated with calf intestinal alkaline phosphatase (CIAP) and then digested with a second restriction enzyme to yield compatible ends. This is a rather convoluted technique but the resultant vector is mono-phosphorylated and allows for directional cloning. They reported a 95% success rate for directional cloning, however their technique requires an ethanol precipitation and still relies on the actions of ligase.
b) "Hetero-stagger cloning" is another ligase-dependent method which requires a total of four PCR primers (Liu 1996). One set of primers is the traditional PCR primer pair and the second set is equivalent to the first, but includes three additional 5'-GGG residues. The DNA is amplified under normal PCR conditions, the products are denatured by heat and then allowed to reanneal slowly by cooling. Reannealing results in the formation of four distinct species. Only 50% of the products are theoretically cloneable, and only 25% of the products would successfully result in directional clones. The only claimed advantage to this technique is that it allows for modem proofreading polymerases to be used during amplification.
c) A variation of the staggered re-annealing technique has also been used which requires only one primer pair (Ailenberg and Silverman 1996).
d) More recently, Gal et al., (1999) have devised a technique called "autosticky PCR" (AS-PCR) (patent application HU9801320). This technique takes advantage of the observation that abasic sites present in DNA can stall DNA polymerases. In this method, PCR primers are designed to contain abasic sites, which stall the amplifying polymerase, resulting in 5'-overhangs thus enabling ligation into a suitably digested vector. The abasic site is produced by the incorporation of tetrahydrofuran, a stable structural analogue of 2-deoxyribose, at the desired position. This method does provide for directional-cloning, but requires non-traditional reagents and an overnight ligation is recommended.

### Ligase-Independent Cloning Methods (LIC)

a) The original ligase PCR-cloning method was described by Shuldiner et al., (1991). Since then, numerous adaptations of this technique have been developed. The technique described here (Temesgen and Eschrich 1996) requires three PCR primers, in which one of the primers contains an additional 24 nucleotides. This process involves two distinct PCR amplifications, thus increasing the probability of introducing PCR errors into the products. However, the linear products can be directly transformed into *E. coli* obviating the need for ligase. Competent *E*. *coli* strain TG2 cells are required, and it is unclear if classical strains such as JM105 or DH5α are able to be substituted. This technique does provide for directional cloning, although the success is related to the PCR parameters in the second PCR step. Any vector can be used in the technique and no restriction enzymes are needed.
b) Garces and Laborda (1995) reported a similar technique only requiring two PCR primers, one of which has a 20bp 5'-extension. The reaction occurs within a single-tube reaction, and can be adapted for use with any vector, but the efficiency is greatly affected by the PCR parameters.

### v) In Vivo Recombination-based Cloning

PCR-cloning is traditionally completed within the test-tube environment of the laboratory, however, there are at least two reports of cloning using *in vivo* systems. The following technique was based on the observation that when yeast were cotransfected with a linear template and a gapped plasmid, homologous recombination was able to "patch" the two species together (Guthrie and Fink, 1991). PCR products have since been cloned in yeast using this method (Scharer and Iggo 1992). A similar phenomenon has been reported in *E. coli* (Oliner, Kinzler et al. 1993). This technique presumably takes advantage of endogenous exonuclease or polymerase activities encoded by the host, but there is no speculation as to what is exactly occurring. The PCR primers are designed to contain 5'-sequences which are identical to sequences adjacent to a chosen RE site. The linearized vector and the PCR products are cotransfected into *E*. *coli* strain JC8679. This technique may not be suitable for use with traditional *E. coli* strains because independent reports indicate that DH5α cells cannot catalyze intramolecular gap repair, and thus might not be expected to catalyze intermolecular recombination (Hanahan, 1985). A similar methodology described by Bubeck et al., (1993) reported successful recombination in DH5α cells but only if they were transformed by CaCl₂ methods. Two more commonly used techniques for bacterial transformation known as heat shock and electroporation were unsuccessfully used in the above experiment.

### vi) Exonuclease-based PCR cloning

A completely different approach to the cloning of PCR fragments involves the generation of single-strand overhangs through the action of various exonucleases. All of the exo-based methods are ligase-independent and are based on the technique originally reported by Aslanidis and deJong, (1990). Numerous modifications to this technique have allowed for improvements in the method (Haun, Serventi et al. 1992; Kuijper, Wiren et al. 1992), both of which use the 3'-5' exonuclease activity of T4 DNA polymerase. PCR primers are designed to contain a 5'-extension complementary to sequences adjacent to a chosen RE site within the vector. Single-strand overhangs are generated through the exonucleolytic digestion by T4 pol and annealing of single-strand regions between the vector and insert is sufficiently stable to allow for direct bacterial transformation. These techniques require delicate control of incubation periods, as these enzymes are extremely efficient, and if one is not careful, excess DNA can be digested. Kuijper et al., (1992) also reported that there is great variation between enzyme preparations, therefore, requiring fine-tuning of temporal conditions with each new batch of enzyme. A second drawback to these specific methods is the requirement for the addition of dTTPs or dATPs in the exo reaction to stop the enzyme at the appropriate positions.

A similar method to that reported above uses a different enzyme called exonuclease III (Hsiao 1993), which was originally used for cloning in 1992 (Kaluz, Kolble et al. 1992). Its limitation is that only blunt-ended or 5'-overhanging substrates can be efficiently cloned. Substrates with 3'-overhangs cannot be cloned by this method.

More recently, phage T7 Gene6 exonuclease has been used for PCR-cloning (Zhou and Hatahet 1995). In this technique PCR primers are designed to include internal phophorothioate bonds positioned towards the center of the primers. The 3'end of the primers are standard PCR primers, whereas the 5' ends are designed to be complementary to sequences adjacent to a certain RE site. This method produces directional clones and is ligase-independent but requires the use of non-standard PCR primers. US 5,580,759 (Yang, et al.) also discloses a method of construction of recombinant DNA by exonuclease recession.

In summary, a wide variety of methods exist for the cloning of PCR products, and each has its advantage and disadvantages. There remains a need for an optimal cloning method having the following characteristics:
- compatible with the use of any vector and any restriction enzyme;
- requires only two PCR primers comprised solely of natural bases;
- ligase independent;
- time efficient;
- provides almost exclusively directional cloning;
- only the terminal sequences of the amplified region need to be known;
- no possibility of internal digestion of the PCR product;
- any type of amplifying polymerase can be used;
- compatible with various readily available *E. coli* strains;
- transformation of bacteria can be accomplished through a variety of techniques;
- unambiguous selection; and
- adaptable to other techniques such as combinatorial cloning.

### SUMMARY OF THE INVENTION

The present inventors have determined that DNA polymerase, such as vaccinia DNA polymerase, is able to efficiently join one or more linear DNA molecules. The method uses recombination between sequence elements present at the end(s) of a series of DNA molecules to specify the junctions and uses specific DNA polymerases to join the molecules. The method may be used to join one or more linear DNA molecules precisely in a single procedure. The primary application of this new method is in the construction of recombinant DNA molecules, as specifically applied to the cloning of PCR products into any desired vector. The present method offers all of the characteristics of an optimal cloning method listed above.

Accordingly, the present invention provides a single-step method of joining two linear DNA molecules comprising the step of:
- providing a first linear DNA molecule having a first end and a second end;
- providing a second linear DNA molecule having a first end and a second end;
wherein the first linear DNA molecule has a sequence of nucleotides at either the first end or the second end that is complementary to a sequence of nucleotides at either the first end or the second end of the second linear DNA molecule; and
- incubating the two linear DNA molecules in the presence of a poxvirus DNA polymerase which has 3'-5' exonuclease activity under conditions whereby the two linear DNA molecules are joined.

In one embodiment, the present invention provides a method of joining two or more linear DNA molecules comprising the steps of:
- providing two or more linear DNA molecules, each having an x and x' strand, having opposite polarities, wherein the 5' end of the x' strand of one linear DNA molecule has a sequence of nucleotides that is complementary to the 5' end of the x strand of another linear DNA molecule to which it is to be joined; and
- incubating the two or more linear DNA molecules in the presence of a poxvirus DNA polymerase which has 3'-5' exonuclease activity under conditions whereby the two or more linear DNA molecules are joined;
wherein the DNA polymerase has intrinsic exonuclease activity and is capable of performing the DNA joining reaction of the invention.

This method may be used to join any number of DNA molecules. The key is to ensure that the molecules are selected such that the ends of each DNA molecule have complementary regions to the molecule it is to be joined to. Internal molecules must have complementary regions on both ends. Particular applications of this technology are the combinatorial fusion of DNA cassettes and preparation of synthetic genes. The technique is also useful for reconstruction of fragmented clones.

The method of the invention may also be applied to the recircularization of single DNA molecules in which the ends bear substantially complementary nucleic acid sequences. Application of this technique may be in the area of site-directed mutagenesis.

The DNA polymerase that may be used in the method of the invention are all poxvirus DNA polymerises having intrinsic 3'-5' exonuclease activity, that are capable of performing the DNA joining reaction of the invention. Such a polymerase may be identified by assaying for its ability to join two linear DNA molecules, having ends with complementary nucleotide sequences, as described herein. Preferably, the DNA polymerase is vaccinia virus DNA polymerase. The length of the complementary nucleotide sequence on each linear DNA molecule may be between about 5 and about 100 nucleotides, preferably between about 8 and about 50 nucleotides and, most preferably, between about 10 and 35 nucleotides. Various, well known stimulatory factors may be added to the incubation mixture. Such stimulatory factors are those that increase the efficiency of the reaction and stabilize the desired reaction products, for example, single strand DNA binding proteins.

In another of its embodiments, the present invention involves a method of constructing a recombinant DNA molecule comprising the steps of:
- providing a linearized vector DNA molecule and a template DNA molecule, each having a first and a second end;
- providing a first primer DNA molecule having a 5' end that comprises nucleotide sequences that will incorporate nucleotide sequences that are complementary to the first end of the linearized vector molecule onto the first end of the template DNA molecule and a 3' end that is designed to hybridize to a suitable location on the first end of the template DNA molecule;
- providing a second primer DNA molecule having a 5' end that comprises nucleotide sequences that will incorporate nucleotide sequences that are complementary to the second end of the linearized vector molecule onto the second end of the template DNA molecule and a 3' end that is designed to hybridize to a suitable location on the second end of the template DNA molecule;
- amplifying the template DNA molecule using the polymerase chain reaction with the first and second primers to generate a PCR amplified product; and
- incubating the PCR amplified product with the linearized vector DNA molecule in the presence of a poxvirus DNA polymerase to generate a recombinant DNA molecule;
wherein the poxvirus DNA polymerase has intrinsic 3'-5' exonuclease activity and is capable of performing the DNA joining reaction of the invention.

The DNA polymerases that may be used in the method of the invention include all poxvirus DNA polymerases having intrinsic exonuclease activity, preferably 3'-5' exonuclease activity, that are capable of performing the DNA joining reaction of the invention. Such polymerases may be identified by assaying for the ability to join two linear DNA molecules having ends with complementary nucleotide sequences, as described herein. Preferably, the DNA polymerase is vaccinia virus DNA polymerase. Various, well known stimulatory factors may be added to the incubation mixture. Such stimulatory factors are those that increase the efficiency of the reaction and stabilize the desired reaction products, for example, single strand DNA binding proteins.

The number of complementary nucleotides that are incorporated onto the first and second ends of the template DNA molecule may be between about 5 and about 100 nucleotides, preferably between about 8 and about 50 nucleotides and, most preferably, between about 10 and 35 nucleotides.

This method could be readily applied to the insertion of two or more DNA molecules into a vector. Each additional molecule would require two further bipartite PCR primers, each having a first portion that primes a PCR extension that is complementary to the appropriate end of the adjacent molecule and a second portion that is complementary to the appropriate end of the DNA molecule to be amplified.

The linear DNA molecule to be inserted into a linearized vector using the method of the invention need not be obtained through PCR amplification. Therefore the present invention further involves a method of constructing a recombinant DNA molecule comprising:
- providing a linearized vector DNA molecule;
- providing a linear insert DNA molecule having ends that are substantially complementary to the ends of the linearized vector DNA molecule to which they are to be joined; and
- incubating the linearized vector DNA molecule and linear insert DNA molecule in the presence of a Poxvirus DNA polymerase under conditions where the two DNA molecules are joined;
wherein the Poxvirus DNA polymerase has intrinsic 3'-5' exonuclease activity and joins the DNA molecules. The linear insert DNA molecule may be obtained by any known means, for example, chemical synthesis, ligation or annealing.

The present invention provides a method to directionally clone any template DNA molecule into a single restriction site of any vector. The vector ends may be generated from any restriction enzyme cleavage. The method does not require a ligation step nor the use of carefully controlled conditions as is required with methods involving specific exonucleases alone.

The present invention is also directed to kits containing reagents for conducting the method.

These and other aspects of the present invention will be described in greater detail hereinbelow.

### BRIEF DESCRIPTION OF THE DRAWINGS

The features of the present invention will become more apparent from the following description in which reference is made to the appended drawings in which:
Figure I shows a schematic representation (Panel A) and the experimental results (Panels B and C) for the joining of two linear DNA molecules using the method of the invention.
Figure 2 shows a schematic representation (Panel A) and the experimental results (Panel B) for the joining of three linear DNA molecules using the method of the invention.
Figure 3 shows a schematic representation of the application of the method of the invention to the combinatorial fusion of DNA cassettes.
Figure 4 is a schematic showing the application of the method of the invention to the cloning of PCR products.
Figure 5 shows a schematic representation and the experimental results for assays investigating the effect of overlapping and non-overlapping end homology in the DNA joining method of the invention.
Figure 6 is a graph and experimental results (inset) showing the effect of vaccinia single strand binding protein on joint molecule formation using the method of the invention.
Figure 7 provides a schematic representation and experimental results depicting the joining of three linear DNA substrates and the fate of 5'- and 3'-end labels during the joining of three linear DNA molecules using the method of the invention.
Figure 8 provides schematic and experimental results for the cloning of PCR-amplified DNA using the method of the invention.
Figure 9 shows a graphical representation of the effect of homology length on joint molecule formation in the method of the invention.
Figure 10 provides a map of DNA to be recombined to clone a recombinant DNA pRP406-SFV using the method of the invention.
Figure 11 shows the results of *in vitro* recombination of the linear DNA shown in Figure 10 (0.8 % agarose gel).

### DETAILED DESCRIPTION OF THE INVENTION

### Definitions

The term "complementary" as used herein, refers to nucleotide sequences in a single stranded molecule of DNA that are sufficiently complementary to a strand of nucleotide sequences in another DNA molecule to specifically (non-randomly) hybridize to it with consequent hydrogen bonding.

The term "linear DNA molecule" as used herein refers to a double stranded (duplex) nucleic acid molecule comprising two strands of opposite polarity (sense and antisense) of substantially complementary polynucleotides held together by one or more hydrogen bonds between each of the complementary bases present in a base pair of the molecule.

The term ''recombinant DNA molecule" as used herein refers to a hybrid DNA molecule comprising at least two nucleotide sequences not normally found together in nature.

The term "nucleotide" as used herein refers to a monomeric unit of DNA consisting of a sugar moiety (pentose), a phosphate group and a nitrogenous heterocyclic base. The base is linked to the sugar moiety via the glycosidic carbon (1' carbon of pentose) and that combination of base and sugar is a nucleoside. When the nucleoside contains a phosphate group bonded to the 3' or 5' position of the pentose it is referred to as a nucleotide. A sequence of operatively linked nucleotides is typically referred to herein as a "nucleotide sequence'', and their grammatical equivalents, and is represented by a formula whose left to right orientation is in the conventional direction of 5'-terminus to 3'-terminus.

### DNA Joining Methods

The present invention relates to a method to join two or more linear DNA molecules using specific DNA polymerases. The method relies on the ability of specific DNA polymerases to facilitate the recombination of linear DNA molecules having ends that share complementary nucleotides.

Accordingly, the present invention provides a method of joining two linear DNA molecules comprising the step of:
- providing a first linear DNA molecule having a first end and a second end;
- providing a second linear DNA molecule having a first end and a second end,
wherein the first DNA molecule has a sequence of nucleotides at either the first end or the second end that is complementary to a sequence of nucleotides at either the first end or the second end of the second linear DNA molecule; and
- incubating the two linear DNA molecules in the presence of a poxvirus DNA polymerase, which has 3'-5' exonucleose activity under conditions whereby the two linear DNA molecules are joined.

The first and/or second linear DNA molecule can be comprised of one or more linear DNA molecules. For example, the first linear DNA molecule may consist of two linear DNA molecules that have been previously joined using the method of the invention.

In one embodiment, the present invention provides a method of joining two or more linear DNA molecules comprising the steps of:
- providing two or more linear DNA molecules, each having an x and x' strand having opposite polarities, wherein the 5' end of the x' strand of each linear DNA molecule has a sequence of nucleotides that is complementary to a sequence of nucleotides at the 5' end of the x strand of the linear DNA molecule to which it is to be joined; and
- incubating the two or more linear DNA molecules in the presence of a Poxvirus DNA polymerase under conditions whereby the two or more linear DNA molecules are joined;
wherein the Poxvirus DNA polymerase has intrinsic 3'-5' exonuclease activity and is capable of performing the DNA joining reaction of the invention.

DNA polymerases that work in the methods of the invention are those having intrinsic 3'-5' exonuclease activity, that are capable of performing the DNA joining reaction of the invention. Such polymerases may be identified by assaying for the ability to join two linear DNA molecules having ends with complementary nucleotide sequences as described in Experiment 2 herein (Experimental Section). Purified vaccinia DNA polymerase was used in the examples presented herein. The DNA polymerases of the invention are either commercially available or may be prepared using standard recombinant DNA technology. For example, vaccinia DNA polymerase may be purified from vaccinia-infected BSC-40 cells as described in McDonald and Traktman (1994) and Willer, Mann *et al*. (1999). Alternatively, vaccinia DNA polymerase may be purified from cells infected with wild-type vaccinia virus. We assay polymerases to measure their ability to join two linear DNA molecules having ends with complementary nucleotide sequences. The DNA polymerases useful in the invention have intrinsic 3'-5' exonuclease activity.

Various known stimulatory factors may be added to the incubation mixture to enhance the efficiency of the reaction and/or stabilize the reaction products. Suitable stimulatory factors include single strand DNA binding proteins. Some single strand DNA binding proteins that may be used include, but are not limited to, vaccinia and *E. coli* single strand binding proteins, Herpes simplex virus ICP8 protein, and yeast and human replication Protein A (eg. yRPA and hRPA). A skilled person would be able to readily identify other suitable stimulatory factors that may be usefully combined with a DNA polymerase in the methods of the invention. In one aspect of the invention, the vaccinia single strand binding protein is used to enhance the yield of a DNA joining reaction employing vaccinia DNA polymerase.

The degree of complementation between the ends of the linear DNA molecules can vary from between about 5 and about 100 nucleotides, preferably between about 8 and about 50 nucleotides and, most preferably, between about 10 and 35 nucleotides. The linear DNA molecules that may be joined using the method of the invention may be obtained from genomic DNA of prokaryotic or eukaryotic genomes, as well as from various vectors, including plasmids, cosmids, phage, BACs and the like, using restriction enzyme cleavage. Alternatively, the linear DNA molecule may be synthesized by chemical techniques, for example, using automated synthesis and the like. The DNA molecule may also be derived from RNA species, such as mRNA, tRNA and rRNA, from any species and may first be converted to cDNA by reverse transcriptase and the amplified as described in Sambrook *et al*. (1989).

In its most general application, the present invention provides a method to join two or more linear DNA molecules. Figure I shows a schematic of this reaction for the joining of two linear DNA molecules (Panel A). This illustrates how two substrates sharing complementary sequences on the left and right ends can be incubated in the presence of a specific DNA polymerase to form a dimer. Panel C of Figure I shows the results from an experiment where a 1:1 mixture of two substrates, sharing 35 bp of complementary sequence at the left and right ends, was incubated with varying quantities of vaccinia DNA polymerase and the products separated by size using an agarose gel. No reaction is observed when the polymerase is omitted (lane 2) and increasing yields of products were seen when increasing amounts of polymerase were added (lanes 3-9).

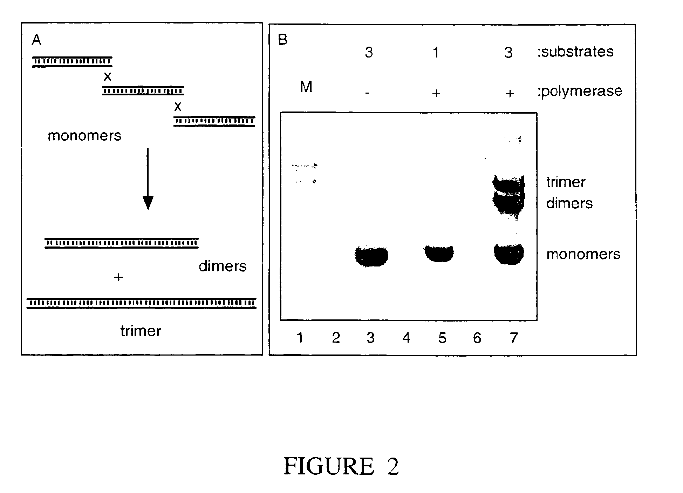

Figure 2, Panel A, shows the joining of three linear DNA molecules to produce a trimer. In this case the "middle" molecule must have sequences of DNA on each end that are complementary to the ends of the two flanking molecules. The results from an actual experiment where the middle molecule had about 20 bp of complementary DNA sequences with the two flanking molecules on each end are shown in Panel B of Figure 2. The results in Panel B of Figure 2, also illustrate an important control (lane 5). The polymerase will not form concatemers when provided with just one of the three substrates, since the ends of identical molecules do not share the appropriate type of complementation. This specificity means that one can direct exactly how two or more molecules are fused, simply by controlling a nearly infinite array of terminal sequence overlaps.

Accordingly, the present invention provides a method of joining three linear DNA molecules comprising:
- providing a first DNA molecule having a first end and a second end;
- providing a second linear DNA molecule having a first end and a second end;
- providing a third linear DNA molecule having a first end and a second end;
wherein the first linear DNA molecule has a sequence of nucleotides at the first end that is complementary to a sequence of nucleotides at either the first or the second end of the second linear DNA molecule and wherein the first linear DNA molecule has a sequence of nucleotides at the second end that is complementay to a sequence of nucleotides at either the first or the second end of the third linear DNA molecule, and
- incubating the three linear DNA molecules in the presence of a Poxvirus DNA polymerase which has intrinsic 3'-5' exonuclease activity under conditions whereby the three linear DNA molecules are joined.

One skilled in the art will appreciate that the above method can be used to join any number of DNA molecules provided the DNA molecules contain the appropriate complementary sequences to join one molecule to another. For example, to join a fourth DNA molecule to the three DNA molecules described above, either the second or third DNA molecule will have a sequence of nucleotides at its free end (i.e. the end that is not joined to the first DNA molecule) that is complementary to a sequence on one end of the fourth DNA molecule.

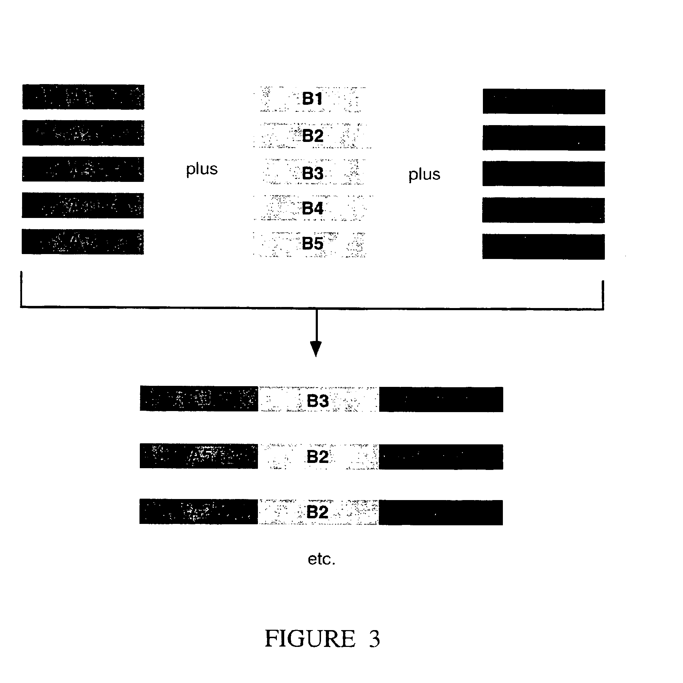

Another application of the present invention is in the combinatorial fusion of DNA cassettes as illustrated in Figure 3. In this example, three different pools of DNA molecules are prepared. Within a pool of molecules, the central portion of each molecule varies in sequence while the ends are identical. For example, as shown in Figure 3, A1-A5 all have the same about 5 to about 100, preferably about 8 to about 50, more preferably about 10 to about 35, nucleotide sequences at the right end, but the remaining portion of the molecules vary in sequence. The left ends of the molecules in the "B" pool are compatible with the right ends of molecules in the "A" pool. Similarly, the left ends of the molecules in the "C'' pool are compatible with the right ends of molecules in the "B" pool. By compatible, it is meant that they share appropriate complementary sequences ranging from about 5 to about 100, preferably about 8 to about 50, more preferably about 10 to about 35, nucleotides. Incubation of the mixture of molecules from pools A, B and C with a DNA polymerase useful in the method of the invention will create trimers composed of one of each of the molecules selected from the A, B, and C pools. The resulting pool of trimers should express a random assortment of "A", "B" and "C" variants. The number of "pools" and molecules within each pool could be varied to potentially produce a random assortment of millions of different DNA molecules. The combinatorial fusion of DNA cassettes using the method of the invention may be applied to the construction of DNA sequences encoding novel proteins through the combinatorial fusion of DNA molecules encoding different protein structural domains. These libraries of proteins may be screened for novel binding specificities.

The method of the invention may also be used to recombine (circularize) a single DNA molecule in which the ends bear substantially complementary nucleic acid sequences. Therefore, the present invention also relates to a method of circularizing linear DNA molecules comprising:
- PCR amplifying a linear DNA molecule in the presence of primers that will introduce substantially complementary nucleic acid sequences onto each end of the linear DNA molecule; and
- incubating the PCR amplified linear DNA molecule in the presence of a Poxvirus DNA polymerase under conditions where the linear DNA molecule is circularized;
wherein the Poxvirus DNA polymerase has intrinsic 3'-5' exonuclease activity and circularizes the DNA molecule.

One use for the application of the method of the invention to the circularization of single DNA molecules is in the area of site directed mutagenesis. For example, a plasmid comprising a gene of interest could be PCR amplified using primers designed to incorporate substantially complementary nucleic acid sequences on each end. If one designed the primers so that mutations are introduced into the plasmid within the gene of interest, mutated forms of the gene may be obtained.

Several additional reaction features were investigated. Perhaps most important is the fact that vaccinia polymerase does not seem to care what type of ends are present on the two recombining molecules. Molecules bearing 5' overhanging, 3' overhanging or blunt ends, in any pairwise combination, were still substrates as long as the two molecules shared appropriate sequence complementation.

### DNA Cloning Methods

In another of its embodiments, the present invention provides a method of cloning any double stranded PCR product into any vector. Therefore there is provided, a method of constructing a recombinant DNA molecule comprising the steps of:
- providing a linearized vector DNA molecule and a template DNA molecule, each having a first and a second end;
- providing a first primer DNA molecule having a 5' end that comprises nucleotide sequences that will incorporate nucleotide sequences that are complementary to the first end of the linearized vector molecule onto the first end of the template DNA molecule and a 3' end that is designed to hybridize to a suitable location on the first end of the template DNA molecule;
- providing a second primer DNA molecule having a 5' end that comprises nucleotide sequences that will incorporate nucleotide sequences that are complementary to the second end of the linearized vector molecule onto the second end of the template DNA molecule and a 3' end that is designed to hybridize to a suitable location on the second end of the template DNA molecule;
- amplifying the template DNA molecule using the polymerase chain reaction with the first and second primers to provide a PCR amplified product; and
- incubating the PCR amplified product with the linearized vector DNA molecule in the presence of a DNA polymerase to generate a recombinant DNA molecule;
wherein the DNA polymerase has intrinsic 3'-5' exonuclease activity and is capable of performing the DNA joining reaction of the invention.

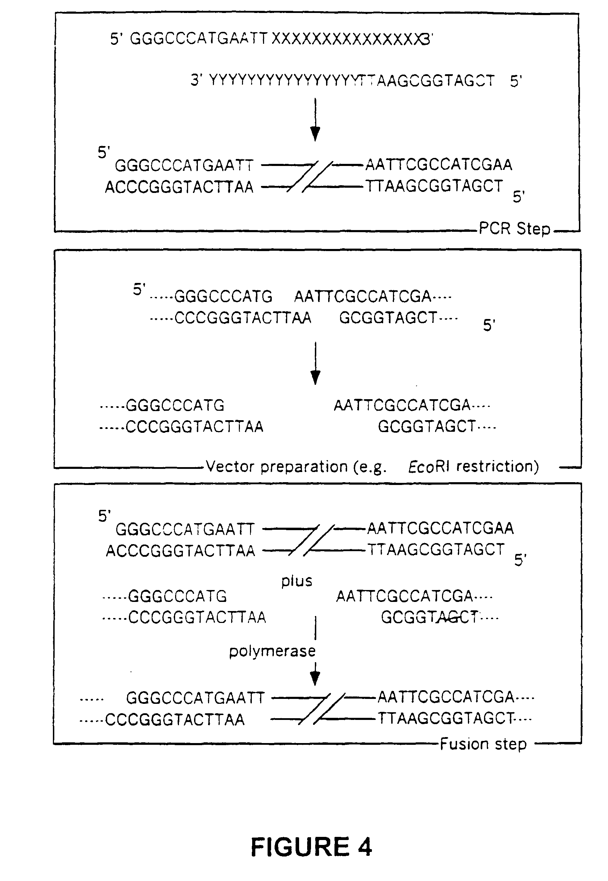

In this method, all that is required is that the PCR primers be extended to incorporate some nucleotides that duplicate the base sequence flanking the chosen cloning site in the vector. The suggested approach is illustrated in Figure 4. A set of two bipartite primers is designed. The 3' end of the first primer molecule is designed to hybridize with the first end of the template DNA molecule, and the 5' end of the first primer molecule has a sequence of from about 5 to about 100, preferably about 8 to about 50, more preferably about 10 to about 35, nucleotides that are designed to incorporate sequences in the final PCR product that are complementary to the first end of the vector DNA molecule. The 3' end of the second primer is designed to hybridize with the second end of the template DNA molecule, and the 5' end of the second primer molecule has a sequence of from about 5 to about 100, preferably about 8 to about 50, more preferably about 10 to about 35, nucleotides that are designed to incorporate sequences in the final PCR product that are complementary to the second end of the vector DNA molecule. The two primers are then annealed to the template DNA molecule which is then PCR amplified using standard conditions to generate a PCR amplified product. The vector is prepared by treating it with the appropriate restriction enzyme to cut it at the chosen insert site using standard conditions.

The term primer as used herein is meant to describe a bipartite primer or a primer having a first and second portion. A first portion of the primer is designed to be complementary to the appropriate end of a template DNA molecule and a second portion of the primer is designed to be complementary to nucleotide sequences on one side of the chosen restriction site of the vector. The bipartite oligonucleotide primers of the present invention may be broadly defined as single stranded oligonucleotides that are complementary to DNA molecules of interest and will allow the DNA molecules to be incorporated into a vector. Bipartite primers will generally have a minimum length of about 18 nucleotides and a maximum length of about 200 nucleotides, preferably about from 25 nucleotides to about 100 nucleotides, more preferably from about 30 nucleotides and about 40 nucleotides. This method could be readily applied to the insertion of two or more DNA molecules into a vector. Each additional molecule would require two further bipartite PCR primers, each having a first portion that primes a PCR extension that is complementary to the appropriate end of the adjacent molecule and a second portion that is complementary to the appropriate end of the DNA molecule to be amplified.

The PCR amplified product and the linearized vector are then incubated in a suitable reaction buffer in the presence of a DNA polymerase having intrinsic 3'-5' exonuclease activity, that is capable of performing the DNA joining reaction of the invention, for about 5 to about 60 minutes, preferably from about 10 to about 40 minutes, most preferably from about 15 to about 30 minutes. Purified vaccinia DNA polymerase was used in the examples presented herein. The reaction buffer may be any buffer that is used in DNA annealing reactions. The temperature may be in the range of from about 35-40 °C, more preferably about 37°C.

The poxvirus DNA polymerases that work in this method may be identified by assaying for the ability to join two linear DNA molecules having ends with appropriate complementary sequences as describe in Example 2 herein (Experimental Section). The DNA polymerases of the invention are either commercially available or may be prepared using standard recombinant DNA technology. For example, vaccinia DNA polymerase may be purified from vaccinia-infected BSC-40 cells as described in McDonald and Traktman (1994) and Willer, Mann *et al*. (1999). Alternatively, vaccinia DNA polymerase may be purified from cells infected with wild-type vaccinia virus. We assay polymerases to measure their ability to join two linear DNA molecules having ends with complementary nucleotide sequences. The poxvirus DNA polymerases useful in the invention have intrinsic 3'-5' exonulcease activity. The invention also extends to cover those DNA polymerases with 5'-3' exonulcease activity.

Various known stimulatory factors may be added to the incubation mixture to enhance the efficiency of the reaction and/or stabilize the reaction products. Suitable stimulatory factors include single strand DNA binding proteins. Some single strand DNA binding proteins that may be used include, but are not limited to, vaccinia and *E. coli* single strand binding proteins, Herpes simplex virus ICP8 protein, and yeast and human replication Protein A (eg. yRPA and hRPA). A skilled person would be able to readily identify other suitable stimulatory factors that may be usefully combined with a DNA polymerase in the methods of the invention. In one aspect of the invention, the vaccinia single strand binding protein is used to enhance the yield of a DNA cloning method of the invention employing vaccinia DNA polymerase.

The method of the invention may be used to PCR clone any variety or number of target DNA molecules. The only limitation on size is the capacity of the vector molecule to carry the insert in transformation and replication in the host cell. Any vector capable of replicating in a prokaryotic or eukaryotic cell is usable with the present invention, such as plasmids, cosmids, phage, BACs and the like. The choice of vector depends on the functional properties desired, for example, protein expression, and the host cell to be transformed. Preferably, the vector has a known sequence of about 5 to about 100, preferably about 8 to about 50, most preferably about 10 to about 35 nucleotides, on either side of the chosen restriction enzyme site. The DNA molecules that may be incorporated into a recombinant DNA molecule using the method of the invention may be obtained from genomic DNA of prokaryotic or eukaryotic genomes, as well as from various vectors, including plasmids, cosmids, phage, BACs and the like, using restriction enzyme cleavage. Alternatively, the DNA molecules may also be synthesized by automated synthesis and the like. The nucleic acid may also be derived from RNA species, such as mRNA, tRNA and rRNA, from any species and may first be converted to cDNA by reverse transcriptase and the amplified as described in Sambrook *et al*. (1989).

The linear DNA molecule to be inserted into a linearized vector using the method of the invention need not be obtained through PCR amplification. Such a molecule may also be prepared by linearization of a circular double strand DNA molecule by restriction enzyme digestion, with subsequent attachment of synthetic oligomer linkers onto the ends, or secondary digestion of an already linear DNA molecule with restriction enzymes, followed by addition of adapter/linkers onto the ends, or by annealing of two appropriately designed synthetic single strand DNA oligomers which can be annealed to form an intact duplex DNA molecule, or any other related method. Incubation of a linear DNA molecule, obtained by such a method, with a linearized vector DNA molecule in the presence of a DNA polymerase, having intrinsic exonuclease activity, will provide the desired recombinant DNA molecule. Therefore the present invention also relates to a method of constructing a recombinant DNA molecule comprising:
- providing a linearized vector DNA molecule;
- providing a linear insert DNA molecule having ends that are substantially complementary to the ends of the linearized vector DNA molecule to which they are to be joined; and
- incubating the linearized vector DNA molecule and linear insert DNA molecule in the presence of a poxvirus DNA polymerase under conditions where the two DNA molecules are joined;
wherein the poxvirus DNA polymerase has intrinsic 3'-5' exonuclease activity and joins the DNA molecules.

### Transformation of Recombinant DNA Molecules

The reaction mixture obtained from the incubation of DNA polymerase with the linearized vector and the PCR amplified product may be used directly to transform any host cell using standard transformation procedures. Therefore the present invention also provides a method to produce a recombinant DNA product comprising the steps of:
- providing a linearized vector DNA molecule and a template DNA molecule, each having a first and a second end;
- providing a first primer DNA molecule having a 5' end that comprises nucleotide sequences that will incorporate nucleotide sequences that are complementary to the first end of the linearized vector molecule onto the first end of the template DNA molecule and a 3' end that is designed to hybridize to a suitable location on the first end of the template DNA molecule;
- providing a second primer DNA molecule having a 5' end that comprises nucleotide sequences that will incorporate nucleotide sequences that are complementary to the second end of the linearized vector molecule onto the second end of the template DNA molecule and a 3' end that is designed to hybridize to a suitable location on the second end of the template DNA molecule;
- providing the template DNA molecule using the polymerase chain reaction with the first and second primers to provide a PCR amplified product;
- incubating the PCR amplified product with the linearized vector DNA molecule in the presence of a DNA polymerase to generate a recombinant DNA molecule;
- transforming the recombinant DNA molecule into a host cell; and
- isolating the recombinant DNA product;
wherein the DNA polymerase has intrinsic 3'-5' exonuclease activity and is capable of performing the DNA joining reaction of the invention. A suitable stimulatory factor may optionally be used in the methods of the invention. For example, one may incubate the PCR amplified product with the linearized vector DNA molecule in the presence of a DNA polymerase and a single strand DNA binding protein to generate a recombinant DNA molecule.

Any variety of cell that is transformable may serve as a host cell, such as *E*. *coli* SURE, JM105, DH5α, HB101, XL1-blue and the like. Other bacterial hosts may include *Bacillus* or *Pseudomonas* species and the like. By way of example, eukaryotic host cells may include *Saccharomyces* species. One of the advantages of the present invention is that the method is selective for the correct orientation of the insert DNA. Therefore, host cells that have been transformed by a recombinant vector will have the insert DNA in the correct orientation in that vector.

The invention extends to cover the transformation of host cells with reaction products obtained from the cloning of any linear DNA molecule into a linearized vector molecule using the method of the invention. The linear DNA molecule to be cloned may be prepared using PCR as described above or by linearization of a circular dsDNA molecule by restriction enzyme digestion, with subsequent attachment of synthetic oligomer linkers onto the ends, or secondary digestion of an already linear DNA molecule with restriction enzymes, followed by addition of adapter/linkers onto the ends, or by annealing of two appropriately designed synthetic ssDNA oligomers which can be annealed to form an intact duplex DNA molecule, or any other related method.

### Kits

The present invention also provides kits suitable for directionally cloning PCR products (or any other linear DNA molecule with appropriate homologous nucleotides on the ends) into a linearized vector. The kit may comprising, in separate containers, an aliquot of a poxvirus DNA polymerase having intrinsic 3'-5' exonuclease activity that is capable of performing the DNA joining reaction of the invention, an aliquot of reaction buffer and a single strand DNA binding protein. An aliquot refers to an amount of the component sufficient to perform at least one program of cloning. The DNA polymerase may be provided as a solution of known concentration, in a buffer optionally comprising a suitable stabilizer, or may be provided as a predetermined aliquot of a freeze-dried product for dissolution in a suitable buffer. The kit also comprises reagents required to perform a positive control reaction. Such reagents include, in separate containers, an aliquot of linearized vector, an aliquot of insert DNA with first and second ends having appropriate complementary sequences, an aliquot of DNA polymerase having intrinsic exonuclease activity that is capable of performing the DNA joining reaction of the invention, optionally, an aliquot of a stimulatory factor such as a single strand DNA binding protein, and an aliquot of reaction buffer.

The present invention will be further illustrated in the following examples. However, it is to be understood that these examples are for illustrative purposes only, and should not be used to limit the scope of the present invention in any manner.

### EXPERIMENTAL EXAMPLES

### Materials and Methods

Linear substrates were prepared by restriction enzyme digestion of plasmid pDW101, followed by phenol extraction and ethanol precipitation. pDW101 derives from pBluescript (KS+) (Stratagene) through a spontaneous deletion of nucleotides 618, 619 and 621. In some experiments, the linear substrates were purified by agarose electrophoresis and extracted from the agarose using a Geneclean II kit (New England Biolabs). [α - ³²P] dCTP and [γ-³²P] dATP were purchased from NEN/Mandel Scientific. End labeled substrates were generated by treatment with shrimp alkaline phosphatase (United States Biochemical, Cleveland), followed by incubation with either T4 polynucleotide kinase and [γ -³²P] dATP (3000 Ci/mmol) or Klenow enzyme and [α - ³²P] dCTP (3000 Ci/mmol). Unincorporated radionucleotides were removed by G-25 spin columns (Pharmacia) and radioactivity was quantitated by Cherenkov (Schneider, 1971). *Hind*III-digested lambda phage DNA and *Hae*III-digested ΦX174 DNA markers were purchased from New England Biolabs. Luciferase assay kit was purchased from Promega.

### Cell and Virus Culture

BSC-40 cells were kindly provided by Dr. E. Niles (SUNY, Buffalo) and grown at 37 °C in DMEM (Gibco BRL) supplemented with 1% nonessential amino acids and 5% fetal calf serum in a 5% CO₂ environment. Vaccinia virus (strain WR) was purchased from the ATCC.

### Experiment 1: Vaccinia DNA Polymerase Purification

Purification of vaccinia DNA polymerase from vaccinia-infected BSC-40 cells has been described (McDonald and Traktman 1994; Willer, *Mann et al*. 1999). Briefly, a crude lysate from 60 150-cm² dishes of BSC40 cells co-infected with vTMPOL and VTF7.5 was subjected to purification through DEAE cellulose, phosphocellulose, ceramic hydroxyapatite and HiTrap heparin columns. Active fractions eluting at each chromatographic step were determined by DNA polymerase assays (Willer, Mann *et al*. 1999). Protein purity was determined by silver staining of denaturing polyacrylamide gels. Protein concentrations were assayed by using a dye-binding assay (Biorad) and a bovine serum albumin standard.

### Experiment 2: Assay for Correct DNA Polymerase Activity

We measure the intrinsic strand joining activity of DNA polymerases, such as those encoded by bacteriophage (e.g. T4, T7), bacteria (e.g. *E. coli),* fungi (e.g. S. *cerevisiae*) and viruses (e.g. poxviruses, herpes viruses, adenoviruses, African swine fever virus and other Iridoviruses, and Bacculoviruses). The polymerases are assayed to measure their activity in the DNA joining reaction by incubating a reaction mixture comprising 30 mM Tris-HCl (pH 7.9), 5 mM MgCl₂, 70 mM NaCl, 1.8 mM dithiothreitol, 88 µg/mL acetylated BSA, 350 ng of each of a first and second linear DNA substrate and the polymerase to be assayed. The first and second linear DNA substrate must have complementary nucleotide sequences on their right and left ends respectively and may be obtained by a variety of methods including chemical synthesis and derivation of nucleic acid fragments from native nucleic acid sequences existing as genes, or parts of genes, in a genome, plasmid, or other vector, such as by restriction endonuclease digest (see, for e.g., preparation of linear substrates described in the Materials and Methods section above). The polymerase has the required activity if the formation of dimers is detected on an agarose gel stained with ethidium bromide. A preferred polymerase will allow the formation of more than about 1% of dimeric products.

### Experiment 3: DNA Joining Reactions

Standard DNA joining reactions (20 µL) contained 30 mM Tris-HCl (pH 7.9), 5 mM MgCl₂, 70 mM NaCl, 1.8 mM dithiothreitol, 88 µg/mL acetylated BSA, 350 ng of each linear substrate, and varying quantities (usually 0.1 µg) of purified vaccinia DNA polymerase. DNA joining reactions employing single strand binding protein also included 25 µg/mL of recombinant vaccinia single strand binding protein (thought to be the IL3 gene product, Tseng *et al*. 1999) in the reaction mix. Reactions were incubated at 37 °C for 20 min, deproteinized and the reaction products were separated through a 0.8% agarose gel as described previously (Zhang and Evans, 1993). Ethidium- stained gels were photographed under UV illumination using Polaroid 665 film. DNA quantitation was determined by desitometry. Joining of Two Linear Substrates:

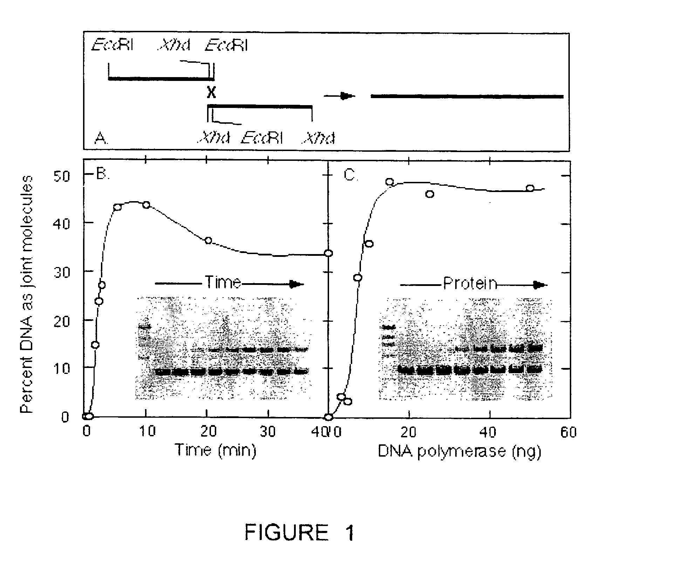

To show that vaccinia DNA polymerase can catalyze strand joining reactions, two linear duplex substrates, sharing a small amount of overlapping sequence homology at the ends of the molecules, were prepared. This was accomplished by cutting pDW101 (SEQ.ID.NO.1) with EcoRI (SEQ.ID.NO.2) and XhoI (SEQ.ID.NO.3) (Fig. 1A), leaving 33 bp of properly oriented sequences. DNA polymerase was then incubated with these DNAs and assayed for concatemer-forming activity using gel electrophoresis. It observed that the polymerase converted 45% of the input DNA into dimeric products over a 10 min period, with a concomitant loss of monomeric molecules (Fig. 1B). The yield of dimer declined slightly thereafter, stabilizing with about 35% of the molecules converted to product. The reaction yield was also dependent upon protein concentration (Fig. 1 C).

### Duplex annealing selectively pairs homologous ends:

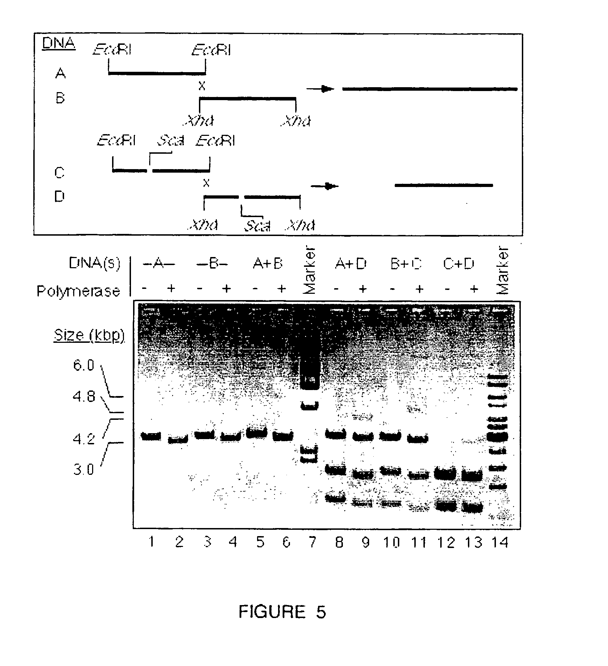

This pairing reaction required two different DNA substrates sharing some small amount of overlapping end homology. Controls showed that the reaction cannot produce joint molecules when the only available substrates were a pool of identical duplexes (Fig. 5, lanes 1-6). To determine which ends of the two substrates were being joined, EcoRI and XhoI cut molecules were further digested with ScaI, producing DNA fragments that can be differentiated by size (Fig. 5, inset). Vaccinia DNA polymerase selectively joined the two molecules sharing a segment of DNA bounded by the XhoI and EcoRI sites, as judged by the appearance of recombinants 4.2, 4.8, and 3.0 kbp in length (Fig. 5, lanes 9, 11, and 13, respectively). Such product sizes are consistent with these molecules being composed of the 3.0+1.2, 3.0 + 1.8, and 1.2 + 1.8 kbp DNA fragments encoding the XhoI-EcoRI interval.

The restriction enzymes used to prepare the interacting ends shown in Figures I and 5 leave 5'-overhanging ends with 4 nt overhangs. To test whether ends also affected reaction efficiency, various substrates were prepared by cutting pDW101 with different restriction enzymes. All possible combinations of molecules bearing 5'-overhanging, blunt, or 5'-recessed ends were then tested to see whether they were still substrates. It was found that vaccinia polymerase has no end preference. There was also no obvious correlation between the yield of duplex product and the length of sequence overlap, when the length of shared end homology ranged from 18 to 84 bp.

### Other reactions:

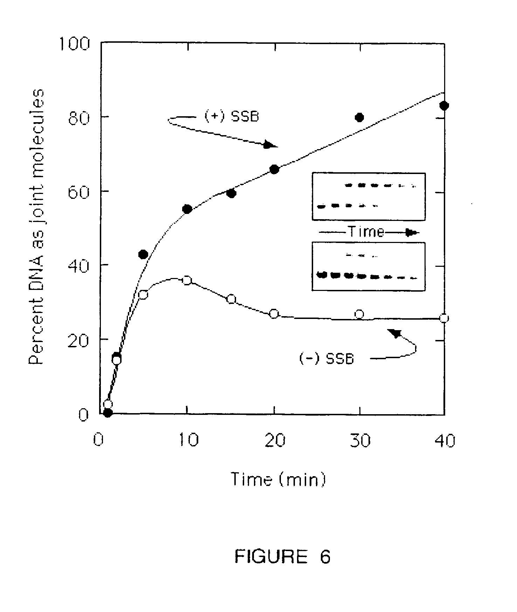

The effect of adding MgCl₂, spermidine, or vaccinia virus single strand binging protein (SSB) on the reaction efficiency was examined using KpnI- and NotI-cut substrates sharing 84 bp of overlapping homology. Magnesium had a reaction optimum of ~10 mM. However, 20 mM MgCl₂ inhibited the reaction, as did spermidine concentrations exceeding 1 mM. The effects of adding recombinant vaccinia SSB (Tseng et al., 1999) were investigated,. The principle replicative high-affinity single-strand DNA binding protein (SSB) is thought to be the I3L gene product (Rochester and Traktman, 1998; Tseng et al., 1999) Vaccinia SSB enhanced the yield of joint molecules 2-3-fold when added at concentrations between 25 and 100 µg/mL (Fig. 6). The net result was that over 80% of the substrate DNAs could be converted to dimers under these optimized reaction conditions.

### Joining of Multiple Substrates:

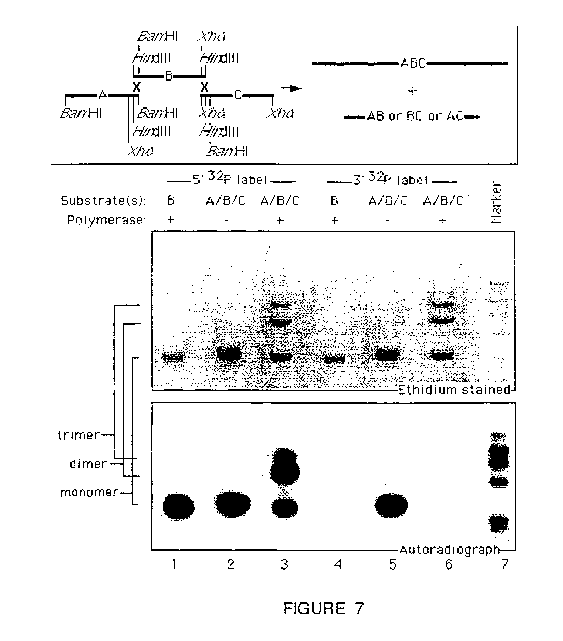

The DNA-joining reaction can also recombine multiple substrates into higher-order concatemers and this can be exploited to provide further insights into the reaction mechanism. The plasmid pDW101 was cut with restriction enzymes so that the "middle" substrate (Fig. 7, molecule "B") shared sequences in common with either end of two additional molecules ("A" and "C"). Alone, the middle duplex was not a substrate for the polymerase catalyzed reaction (Fig. 7, lanes I and 4). However, when the three substrates were incubated together with vaccinia polymerase in standard assays, they were rapidly converted into a mixture of dimers and trimers (Fig. 7, lanes 3 and 6). A small amount of the DNA (~2%) was also converted into higher-order multimers whose structure is uncertain.

For the middle molecule to have been incorporated into a linear trimer, both ends of the molecule must have been subjected to enzymatic processing. To study any modifications which might have been introduced into the ends of these molecules, the HindIII-restricted (SEQ.ID.NO.4) substrate was labeled and monitored the ³²P-label using autoradiography. The ³²P-labels were incorporated into the middle substrates using T4 polynucleotide kinase or Klenow polymerase, to see whether 3' or 5' end-labels suffered different fates. After electrophoresis and ethidium staining, the gel was fixed and autoradiographed to locate the label. It was clear, from inspection of the ethidium-stained gel, that duplex "B" was efficiently incorporated into concatemers regardless of whether it had been labeled on the 3' or on the 5' end (Fig. 7, lanes 3 and 6). However, whereas most of the 5' label was incorporated into trimeric concatemers (Fig. 7, lane 3), none of the 3'-end label was retained in these molecules (Fig. 7, lane 6) or in the unreacted polymerase-treated substrate (Fig. 7, lane 4). These data, plus the MgCl₂ effect, suggests that the vaccinia polymerase 3'-5' exonuclease plays a key role in joint molecule *production in vitro.* Although it is the 3'-5' exonuclease activity of vaccinia DNA polymerase that plays a pivotal roll in the DNA joining reactions described herein, the reaction could work equally as well with DNA polymerases having 5'-3' exonuclease activity.

If this reaction is dependent upon the activity of the 3'-5' exonuclease of vaccinia DNA polymerase, one would predict that dNTPs should inhibit end-joining by inhibiting the nuclease. Experiments confirmed this prediction. Adding 1 µM (total) of all four dNTPs reduced the yield about 50%, while little if any joint molecules were formed in reactions containing >5 µM dNTPs. Dideoxyribonucleotides were also inhibitory, but only at much higher concentrations (>50 µM).

### Example 4: PCR Step

A ~800 bp region from a BamHI clone-derived from Shope Fibroma Virus (Wills, *Delange et al*. 1983; Delange, Macaulay *et al*. 1984) chosen at random was amplified using the PCR primers shown below. Capital letters represent the sequence complementary to the vector (pDW101) and small letters represent the sequence complementary to viral DNA sequence. PCR primers were designed to include complementary sequences from the vector adjacent to the recognition site for the restriction enzyme NotI.
Primer #1: 5' TTCTAGAGCGGCCagaaacaggcatcttacgcgtg 3' (SEQ.ID.NO.5)
Primer #2: 5' TCCACCGCGGTGGCGGCCacggaaacgccttggt 3' (SEQ.ID.NO.6)

### Example 5: Vector Preparation

The vector pDW101 was digested with NotI as depicted below:

### Example 6: Generation of Recombinant Molecules

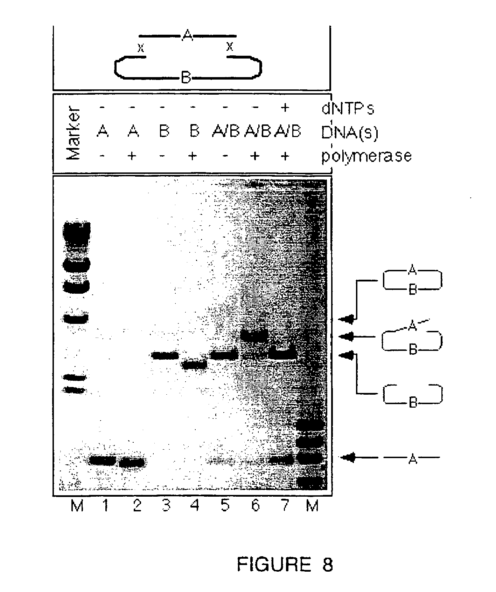

Both the PCR amplified fragment (320 ng) and the linearized vector (600 ng) were incubated together in the presence of purified vaccinia DNA polymerase (150 ng) for 20 minutes as per the annealing assay described in the Materials and Methods. Reaction products were visualized by agarose electrophoresis (Fig. 8). Agarose gels showed that the majority of molecules formed under these conditions were the linear-dimers seen previously (Fig. 8, lane 5). However, because both ends of the PCR-amplified insert shared homology with the vector, a small portion of the reaction products were expected to, and did, migrate at positions characteristic of nicked-circular and higher concatemer forms (Fig. 8, lane 6). None of these reactions occurred in the presence of 5 µM dNTP (Fig. 8, lane 7).

### Example 7: Transformation of Competent Cells

An aliquot (1-2 µL) of the reaction mixture from Example 6 was used to transform three different strains of *E. coli* (SURE, JM105 and DH5α) using standard procedures (SURE and DH5α, by electroporation and JM105, by heat shock). Appropriate dilutions were plated on LB+T plates containing 100 µg/mL ampicillan, 40 µg/mL X-gal (5-bromo-4-chloro-3-indolyl-β-D-galactoside), 0.5 mM IPTG (isopropylthio-β-galactoside) and 50 µg/mL thymine and incubated overnight at 37 °C. DNA was isolated from transformed colonies and sequenced as described previously (Willer, McFadden and Evans, 1999

Table 1 shows data acquired using multiply-recombination deficient SURE cells, although all three strains gave similar results (0.5 - 2 x 10⁵ transformants per µg). White (W) colonies indicate transformation with potentially the correct construct. Blue (B) colonies indicate transformation with the vector substrate alone. It was clear that the yield of stable transformants was greatly dependent upon adding vaccinia DNA polymerase to the reaction mix. Recombinant (white) colonies were the most abundant product (83% of transformants in this particular experiment) even though the NotI-cut vector was not dephosphorylated. Thirteen putative recombinant plasmids were purified and it was observed that all 13 of the plasmids recovered from SURE cells were monomers incorporating a single DNA insert. DNA sequencing showed that all 13 molecules also encoded the insert in the correct orientation and at the expected position. There were 3 base substitutions located within one of the primer-binding sites and 2 other mutations within Taq polymerase-amplified DNA. The fusion points were those expected to be formed through annealing of homologous ends. It was concluded that the concatemers assembled by vaccinia virus polymerase can be processed into stable recombinants with a fidelity comparable to traditional cloning methods.

**TABLE 1**

| Transformation of E. coli with recombinant joint molecules. | | | | |
|---|---|---|---|---|
| | Number of colonies | | Percent | Transformants |
| Reaction | White | Blue | white | per µg (x 10⁻⁵) |
| PCR-amplified insert | | | | |
| (-) polymerase | 2 | 0 | 100 | N/A |
| (+) polymerase | 1 | 0 | 100 | N/A |
| | | | | |
| NotI restricted vector | | | | |
| (-) polymerase | 8 | 1,190 | 0.7 | 4.0 |
| (+) polymerase | 0 | 27 | 0.0 | 0.4 |
| | | | | |
| PCR-amplified insert plus NotI restricted vector | | | | |
| (-) polymerase | 12 | 1,030 | 1.2 | 2.3 |
| (+) polymerase | 1,020 | 210 | 83 | 2.7 |
| (+) polymerase, (+) dNTP | 7 | 220 | 3.1 | 0.5 |

Joint molecules were prepared in reactions containing the indicated reaction components. E. coli SURE cells were electroporated with 1 µL of reaction products. The cells (100 µL or its equivalent) were plated and colonies were counted next day. Cell competency was estimated as 6 x 10¹⁰ transformants per µg using unrestricted pDW101.

### Example 8: The minimal sequence overlap

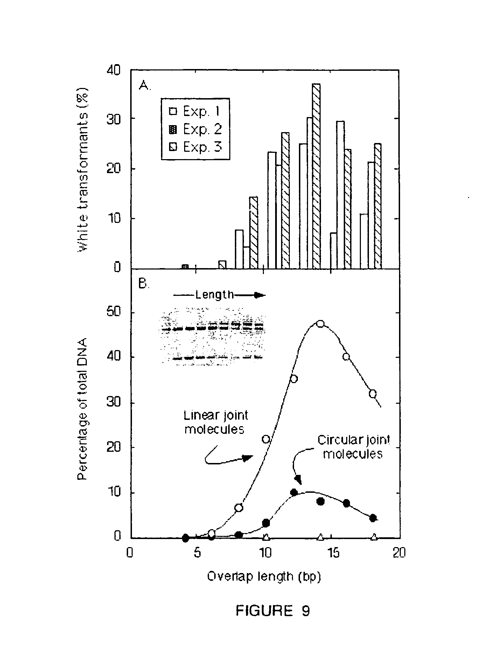

To determine the minimal amount of homology required for production of joint molecules, seven additional primer pairs were synthesized and then the set of eight oligonucleotide pairs were used to again PCR amplify a ~800 bp fragment of SFV DNA. These 14 new PCR primers were similar in structure to the primers described above, except that the amount of sequence homologous to nucleotides flanking the NotI site in a NotI-cut vector now ranged from 4 to 18 bp. These substrates were incubated with vector DNA in optimized assays containing both vaccinia DNA polymerase and gpI3L (which increased the ability to detect otherwise faint circular reaction products) and the yield of both linear and circular joint molecules were quantitated using densitometry. These and other experiments showed that as little as 10 bp of sequence homology between substrates still permitted strand joining by vaccinia polymerase, with a reaction optimum of about 14 bp (Fig. 9, panel B). These reaction products were also used to transform SURE cells, in triplicate, as there was some variation in the absolute efficiency of white transformant production from experiment to experiment. In all of the experiments, the percentage of white (recombinant) bacterial transformants closely paralleled the yield of joint molecules as detected by gel electrophoresis (Fig. 9A).

### Example 9 - Cloning a recombinant plasmid DNA with vaccinia virus DNA polymerase

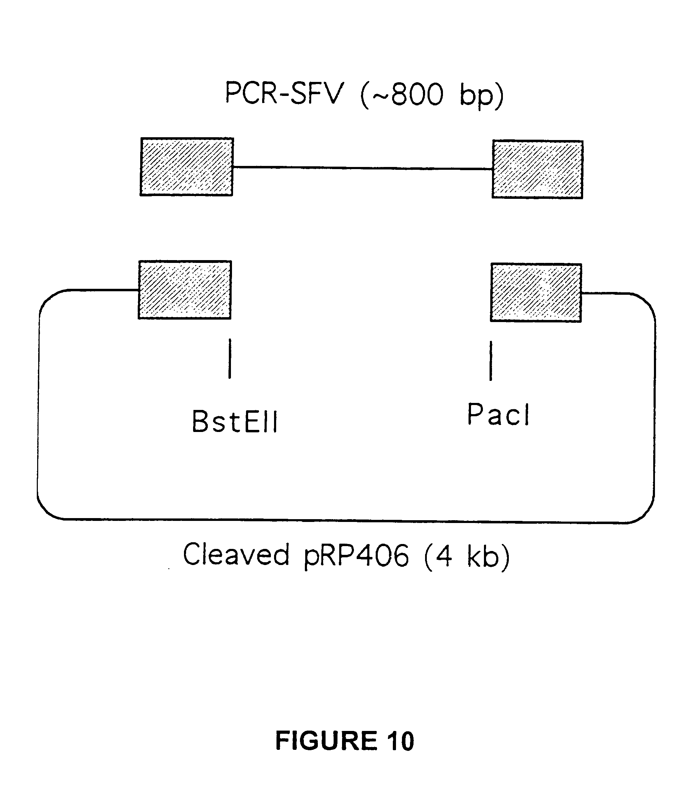

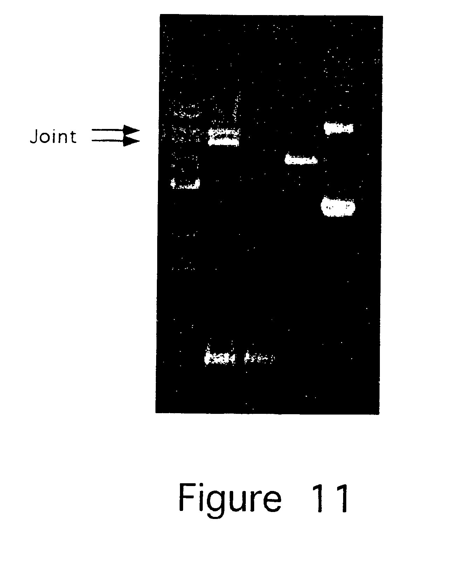

The method of the invention was successfully utilized to clone a recombinant DNA pRP406-SFV (Figure 10, 11).

The cloning method of the present invention was compared with a traditional cloning method. Overall, the method of the present invention was simpler and quicker (Table 2).

Vector: pRP406 (SEQ.ID.NO.8) was cut with Pacl and BstEII and the larger fragment was purified to eliminate the smaller, intervening fragment.

Insert: PCR-SFV(~800 bp, SEQ.ID.NO.9),the polymerase chain reaction was used to amplify a fragment of Shope fibroma virus DNA. PCR primers added 16 bp sequences at the ends of the DNA that were homologous to sequences located at the ends of the cut vector (Fig. 10). Primers used when recombining DNA with pRP406 are shown in SEQ.ID.NOs.10 and 11.

**Table 2 Comparison of Cloning Strategies**

| | Joining by v DNA pol | Traditional method with ligase |
|---|---|---|
| P C R - amplified insert | With >12 nt homology with linear vector at both ends | Cleaved with Bst*EII* and Pac I to generate the sticky ends |
| | 300 ng, 600 fmol | 150 ng, 300 fmol |
| Linear vector | 250 ng, 100 fmol | 100 ng, 40 fmol |
| Reaction condition | v pol 100 ng, v SSB 500 ng in 20 ul 37 °C 10 min | T4 DNA ligase 5 U in 10 ul 20 °C 2 hr |
| Transformation | 1 of 20^{th} of reaction solution into E.coli SURE | 1 of 10^{th} of reaction solution into E.coli SURE |
| Recombinant frequency | 4.8 x 10⁴ / ug vector | 9.2 x 10⁴ / ug vector |

### FULL CITATIONS FOR REFERENCES REFERRED TO IN THE SPECIFICATION

Ailenberg, M. and M. Silverman (1996). "Description of a one step staggered reannealing method for directional cloning of PCR-generated DNA using sticky-end ligation without employing restriction enzymes." Biochem Mol Biol Int 39(4): 771-9.
Aguado, B., Selmes, I. P., and Smith, G. L. (1992). Nucleotide sequence of 21.8 kbp of variola major virus strain Harvey and comparison with vaccinia virus. *J Gen Virol* 73, 2887-902.
Aslanidis, C. and P. J. de Jong (1990). "Ligation-independent cloning of PCR products (LIC-PCR)." Nucleic Acids Res 18(20): 6069-74.
Ball, L. A. (1987). High-frequency homologous recombination in vaccinia virus DNA. *J Virol* 61, 1788-95.
Bubeck, P., M. Winkler, et al. (1993). "Rapid cloning by homologous recombination in vivo." Nucleic Acids Res 21(15): 3601-2.
Cha, J., W. Bishai, et al. (1993). "New vectors for direct cloning of PCR products [published erratum appears in Gene 1994 Apr 8;141(1):149]." Gene 136(1-2): 369-70.
Challberg, M. D., and Englund, P. T. (1979). Purification and properties of the deoxyribonucleic acid polymerase induced by vaccinia virus. *J Biol Chem* 254, 7812-9.
Clark, J. M. (1988). "Novel non-templated nucleotide addition reactions catalyzed by procaryotic and eucaryotic DNA polymerases." Nucleic Acids Res 16(20): 9677-86.
Colinas, R. J., Condit, R. C., and Paoletti, E. (1990). Extrachromosomal recombination in vaccinia-infected cells requires a functional DNA polymerase participating at a level other than DNA replication. *Virus Res* 18, 49-70.
Costa, G. L. and M. P. Weiner (1994). "Polishing with T4 or Pfu polymerase increases the efficiency of cloning of PCR fragments." Nucleic Acids Res 22(12): 2423.
Du, S.. and Traktman. P. (1996). Vaccinia virus DNA replication: two hundred base pairs of telomeric sequence confer optimal replication efficiency on minichromosome templates. *Proc Natl Acad Sci U S A* 93, 9693-8.
DeLange, A. M. (1989). Identification of temperature-sensitive mutants of vaccinia virus that are defective in conversion of concatemeric replicative intermediates to the mature linear DNA genome. *J Virol* 63, 2437-44.
DeLange, A. M., and McFadden, G. (1986). Sequence-nonspecific replication of transfected plasmid DNA in poxvirus- infected cells. *Proc Natl Acad Sci U* S A 83, 614-8.
Delange, A. M., C. Macaulay, *et al*. (1984). "Tumorigenic poxviruses: construction of the composite map of the Shope fibroma virus genome." J. Virol **50**(2):408-416.
Duncan, B. K. and J. A. Chambers (1984). "The cloning and overproduction of Escherichia coli uracil-DNA glycosylase." Gene 28(2): 211-9.
Evans, D. H., Stuart, D., and McFadden, G. (1988). High levels of genetic recombination among cotransfected plasmid DNAs in poxvirus-infected mammalian cells. *J Virol* 62, 367-75.
Fisher, C., Parks, R. J., Lauzon, M. L., and Evans, D. H. (1991). Heteroduplex DNA formation is associated with replication and recombination in poxvirus-infected cells. *Genetics* 129, 7-18.
Gal, J., R. Schnell, et al. (1999). "Directional cloning of native PCR products with preformed sticky ends (autosticky PCR)." Mol Gen Genet 260(6): 569-73.
Garces, C. and J. Laborda (1995). "Single-step, ligase-free cloning of polymerase chain reaction products into any restriction site of any DNA plasmid." Anal Biochem 230(1): 178-80.
Guthrie, C. and Fink, G. (1991). Methods in Enzymology. 194.
Hanahan, D. (1985). In: DNA Cloning, Vol I. Glover, D.M. (ed.), IRL Press, Oxford.
Haun, R. S., I. M. Serventi, et al. (1992). "Rapid, reliable ligation-independent cloning of PCR products using modified plasmid vectors." Biotechniques 13(4):515-8.
Hendricks, S. P., and Mathews, C. K. (1998). Allosteric regulation of vaccinia virus ribonucleotide reductase, analyzed by simultaneous monitoring of its four activities. *J Biol Chem* 273, 29512-8.
Hengen, P. N. (1995). "Methods and reagents. Cloning PCR products using T-vectors." Trends Biochem Sci 20(2): 85-6.
Howell, M. L., Roseman, N. A., Slabaugh, M. B.. and Mathews, C. K. (1993). Vaccinia virus ribonucleotide reductase. Correlation between deoxyribonucleotide supply and demand. *J Biol Chem* 268, 7155-62.
Hsiao, K. (1993). "Exonuclease III induced ligase-free directional subcloning of PCR products." Nucleic Acids Res 21(23): 5528-9.
Ichihara, Y. and Y. Kurosawa (1993). "Construction of new T vectors for direct cloning of PCR products." Gene 130(1): 153-4.
Johnson, G. P., Goebel, S. J., and Paoletti, E. (1993). An update on the vaccinia virus genome. *Virology* 196, 381-401.
Kaluz, S. and A. P. Flint (1994). "Ligation-independent cloning of PCR products with primers containing nonbase residues." Nucleic Acids Res 22(22): 4845.
Kaluz, S., K. Kolble, et al. (1992). "Directional cloning of PCR products using exonuclease III." Nucleic Acids Res 20(16): 4369-70.
Karakousis, G., Ye, N., Li, Z., Chiu, S. K., Reddy, G., and Radding, C. M. (1998). The beta protein of phage lambda binds preferentially to an intermediate in DNA renaturation. *J Mol Biol* 276, 721-31.
Kaufman, D. L. and G. A. Evans (1990). "Restriction endonuclease cleavage at the termini of PCR products [published erratum appears in Biotechniques 1990 Dec;9(6):720]." Biotechniques 9(3): 304, 306.
Kovalic, D., J. H. Kwak, et al. (1991). "General method for direct cloning of DNA fragments generated by the polymerase chain reaction." Nucleic Acids Res 19(16): 4560.
Kuijper, J. L., K. M. Wiren, et al. (1992). "Functional cloning vectors for use in directional cDNA cloning using cohesive ends produced with T4 DNA polymerase." Gene 112(2): 147-55.
Li, Z., Karakousis, G., Chiu, S. K., Reddy, G., and Radding, C. M. (1998). The beta protein of phage lambda promotes strand exchange. J *Mol Biol* 276, 733-44.
Liu, Z. (1996). "Hetero-stagger cloning: efficient and rapid cloning of PCR products." Nucleic Acids Res 24(12): 2458-9.
Liu, Z. G. and L. M. Schwartz (1992). "An efficient method for blunt-end ligation of PCR products." Biotechniques 12(1): 28, 30.
Longo, M. C., M. S. Berninger, et al. (1990). "Use of uracil DNA glycosylase to control carry-over contamination in polymerase chain reactions.'' Gene 93(1): 125-8.
McDonald, W. F. and Traktman, P. (1994). "Analysis of the complete genome of smallpox variola major virus strain Bangladesh-1975." Virology **201**(2): 215-240.
McFadden. G., and Dales, S. (1979). Biogenesis of poxviruses: mirror-image deletions in vaccinia virus DNA. *Cell 18,* 101-8.
McDonald, W. F., and Traktman, P. (1994). Overexpression and purification of the vaccinia virus DNA polymerase. *Protein Expr Purif* 5, 409-21.
Mead, D. A., N. K. Pey, et al. (1991). "A universal method for the direct cloning of PCR amplified nucleic acid." Biotechnology (N Y) 9(7): 657-63.
Merchlinsky, M. (1989). Intramolecular homologous recombination in cells infected with temperature-sensitive mutants of vaccinia virus. *J Virol* 63, 2030-5.
Moss, B. (1996). Poxviridae: The viruses and their replication. 3 rd. ed. *In* "Fundamental Virology" (B. N. Fields, Knipe, D.M., Howley, P.M., Ed.), pp. 1163-1197. Raven, Philadelphia.
Mole, S. E., R. D. Iggo, et al. (1989). "Using the polymerase chain reaction to modify expression plasmids for epitope mapping." Nucleic Acids Res 17(8): 3319.
Moyer, R. W., and Graves, R. L. (1981). The mechanism of cytoplasmic orthopoxvirus DNA replication. *Cell* 27, 391-401.
Mullis, K. B. and F. A. Faloona (1987). "Specific synthesis of DNA in vitro via a polymerase-catalyzed chain reaction." Methods Enzvmol 155: 335-50.
Oliner, J. D., K. W. Kinzler, et al. (1993). "In vivo cloning of PCR products in E. coli." Nucleic Acids Res 21(22): 192-7.
Paques, F., and Haber, J. E. (1999). Multiple pathways of recombination induced by double-strand breaks in Saccharomyces cerevisiae. *Microbiol Mol Biol Rev* 63, 349-404.
Parks, R. J., and Evans, D. H. (1991). Effect of marker distance and orientation on recombinant formation in poxvirus-infected cells. *J Virol* 65, 1263-72.
Pickup. D. J., Bastia, D., Stone, H. O., and Joklik, W. K. (1982). Sequence of terminal regions of cowpox virus DNA: arrangement of repeated and unique sequence elements. *Proc Natl Acad Sci U S A* 79. 7112-6.
Rashtchian, A., G. W. Buchman, et al. (1992). "Uracil DNA glycosylase-mediated cloning of polymerase chain reaction- amplified DNA: application to genomic and cDNA cloning." Anal Biochem 206(1): 91-7.
Rochester, S. C., and Traktman, P. (1998). Characterization of the single-stranded DNA binding protein encoded by the vaccinia virus 13 gene. *J Virol* 72, 2917-26.
Rosemond-Hornbeak, H., and Moss, B. (1974). Single-stranded deoxyribonucleic acid-specific nuclease from vaccinia virus. Endonucleolytic and exonucleolytic activities. *J Biol Chem* 249, 3292-6.
Saiki, R. K., D. H. Gelfand, et al. (1988). "Primer-directed enzymatic amplification of DNA with a thermostable DNA polymerase." Science 239(4839): 487-91.
Sambrook et al. (1989). " Molecular cloning: A laboratory manual." Cold Spring Harbour, N.Y.
Scharer, E. and R. Iggo (1992). "Mammalian p53 can function as a transcription factor in yeast." Nucleic Acids Res 20(7): 1539-45.
Scharf, S.J. (1990). In Innis, M.A., Gelfand, D.H., Sninsky, J.J., and White, T.J. (Eds.), PCR Protocols. Academic Press, New York, pp. 84-91.
Schneider, P.B. (1971). "Determination of specific activity of 32P-labeled compounds using Cernkov counting." J Nucl Med 12(1): 14-16.
Shchelkunov, S. N., and Totmenin, A. V. (1995). Two types of deletions in orthopoxvirus genomes. *Virus Genes* 9, 231-45.
Shuldiner, A. R., K. Tanner, et al. (1991). "Ligase-free subcloning: a versatile method to subclone polymerase chain reaction (PCR) products in a single day." Anal Biochem 194(1): 9-15.
Shuman, S. (1991). "Site-specific DNA cleavage by vaccinia virus DNA topoisomerase I. Role of nucleotide sequence and DNA secondary structure." J Biol Chem 266(3): 1796-803.
Shuman, S. (1992). "DNA strand transfer reactions catalyzed by vaccinia topoisomerase I." J Biol Chem 267(12): 8620-7.
Shuman, S. (1992). "Two classes of DNA end-joining reactions catalyzed by vaccinia topoisomerase I.'' J Biol Chem 267(24): 16755-8.
Shuman, S. (1994). "Novel approach to molecular cloning and polynucleotide synthesis using vaccinia DNA topoisomerase." J Biol Chem 269(51): 32678-84.
Shuman, S. and J. Prescott (1990). "Specific DNA cleavage and binding by vaccinia virus DNA topoisomerase I." J Biol Chem 265(29): 17826-36.
Stahl, M. M., Thomason, L., Poteete, A. R., Tarkowski, T., Kuzminov, A., and Stahl, F. W. (1997). Annealing vs. invasion in phage lambda recombination. *Genetics* 147, 961-77.
Stivers, J. T., S. Shuman, et al. (1994). "Vaccinia DNA topoisomerase I: single-turnover and steady-state kinetic analysis of the DNA strand cleavage and ligation reactions." Biochemistry 33(1): 327-39.
Temesgen, B. and K. Eschrich (1996). "Simplified method for ligase-free cloning of PCR products." Biotechniques 21(5): 828, 830, 832.
Traktman, P. (1990). The enzymology of poxvirus DNA replication. *Curr Top Microbiol Immunol* 163, 93-123.
Tseng, M., Palaniyar, N., Zhang, W., and Evans, D. H. (1999). DNA binding, aggregation, and annealing properties of the vaccinia virus I3L gene product. *J Biol Chem* 274, 21637-44.
Varshney, U. and J. H. van de Sande (1991). "Specificities and kinetics of uracil excision from uracil-containing DNA oligomers by Escherichia coli uracil DNA glycosylase." Biochemistry 30(16): 4055-61.
Weiner, M. P. (1993). "Directional cloning of blunt-ended PCR products." Biotechniques 15(3): 502-5.
Willer, D. O., Mann, M. J. *et al.* (1999). "Vaccinia virus DNA polymerase promotes DNA pairing and strand transfer reactions." Virology **257**(2):511-523.
Willer, D. O., McFadden, G., and Evans, D. H. (1999). The complete genome sequence of shope (rabbit) fibroma virus. *Virology* 264, 319-43.
Wills, A., Delange, A. M. *et al.* (1983). "Physical characterization and molecular
Zhang, W. and Evans, D. H. (1993). "DNA strand exchange catalyzed by proteins from vaccinia virus-infected cells." J. Virol **67**(1):204-212.
Zhou, M. and Z. Hatahet (1995). "An improved ligase-free method for directional subcloning of PCR amplified DNA." Nucleic Acids Res 23(6): 1089-90.

### DETAILED LEGENDS FOR FIGURES

Figure 1. DNA annealing catalyzed by vaccinia DNA polymerase. pDW101 was cut with EcoRI or XhoI to produce 2.96 kbp molecules sharing 33 bp of overlapping sequence homology (Panel A.). Annealing reactions (20 µL) were then prepared containing 0.35 µg of each DNA substrate plus 0.1 µg of vaccinia virus polymerase. After incubation for the indicated times, the reaction products were fractionated using agarose gel electrophoresis and visualized by ethidium bromide staining (Panel B). Alternatively. reactions were prepared containing 0.35 µg of each DNA substrate plus the indicated quantities of DNA polymerase and incubated for 20 min at 37° (Panel C.). A 6 kbp reaction product is formed in Panels B and C.
Figure 2. Trimer formation. Panel A shows a schematic of the reaction. Note that joining three linear molecules is essentially the same reaction needed to join an insert DNA to the two flanking arms of a vector molecule. Panel B shows the reaction products analyzed on an ethidium-stained agarose gel. No joint molecules are formed when only one of the three substrates are provided to the polymerase. This shows that the reaction requires correct complementary sequences at the ends of the reacting molecules.
Figure 3. Combinatorial fusion of DNA cassettes. In this simple scheme three different pools of DNA duplexes are prepared. Within a pool of molecules, the central portion of each molecule varies in sequence while the ends are identical. For example, A1-A5 all have the same ~20 base sequence at the right end but the middle portions of these molecules vary in sequence. The left ends of molecules in the "B" pool are compatible with the right ends of molecules in the "A" pool. Similarily, the left ends of molecules in the "C'' pool are compatible with the right ends of molecules in the "B" pool. Reaction with vaccinia polymerase will create trimers composed of one each of molecules selected from the "A", "B", and "C" pools. The resulting pool of trimers should express a random assortment of "A", "B", and "C" variants.
Figure 4. Proposed cloning method. The PCR primers encode 10-20 additional nucleotides complementary to sequences flanking the vector insertion site.
Figure 5. Substrate requirements. Reactions were prepared containing the indicated substrate DNAs plus or minus vaccinia DNA polymerase (0.1 µg) in 20 µL. The polymerase will not promote joint molecule formation unless two substrates are provided which share some overlapping end homology (lanes 1-6). When the DNA fragments can be differentiated by size, the reaction products comprise DNAs encoding the XhoI - EcoRI overlap region (lanes 8-13).
Figure 6. Effect of vaccinia SSB (gpI3L) on joint molecule formation. Standard reactions (20 µL) contained 0.1 µg of vaccinia virus DNA polymerase (E) or 0.1 µg of polymerase plus 0.5 µg of vaccinia SSB (•). The reactions were incubated for the indicated time, stopped, and the yield of joint molecules determined by gel electrophoresis (inset) and densitometry. Vaccinia SSB increased the extent of the reaction and stabilized the products.
Figure 7. Trimer formation catalyzed by vaccinia virus DNA polymerase. Three substrates were prepared as indicated in the upper panel, and the middle substrate ("B") labeled using polynucleotide kinase or Klenow polymerase. Reactions were prepared as indicated, incubated at 37°C for 20 min, and then fractionated using agarose gel electrophoresis. The reaction products were first visualized with ethidium bromide, and then by autoradiography. No joint molecules were formed in reactions containing polymerase plus only a single DNA substrate (lanes 1 and 4). Only a 5' label survived incubation with the polymerase and was incorporated into dimer and trimer molecules (lane 3).
Figure 8. Cloning of PCR-amplified DNA using vaccinia virus DNA polymerase. An ~800 bp PCR-amplified DNA fragment ("A") was incubated with or without NotI-linearized pDW101 ("B") and vaccinia virus DNA polymerase, as indicated. The reaction products were separated by electrophoresis and visualized with ethidium bromide. Joint molecules were seen only in lane 6. Most of these joint molecules migrated at a position expected of linear dimers (lane 6), but a small portion of the reaction product migrated at a position typical of nicked circular molecules (arrowed). Adding 5 µM dNTPs blocked the reaction completely (lane 7). Table 1 summarizes the effect of transforming E. coli with these reaction products.
Figure 9. Effect of homology length on joint molecule formation. PCR amplified DNAs were prepared containing 4, 6, 8, ..., 18 bp of end sequence identical to sequences found flanking the NotI site in a pBluescript vector. A mixture of PCR amplified DNA plus NotI-cut vector was incubated with 0.1 µg of vaccinia DNA polymerase and 0.5 µg of gpI3L for 10 min and then separated by agarose gel electrophoresis (Panel B, inset). The yield of linear-duplex (○) and circular (•) joint molecules in each reaction was determined by densitometry (Panel B). Omitting the polymerase yielded no joint molecules (Δ). The reaction products (plus joint molecules prepared in two additional experiments) were used to transform bacterial SURE cells and the yield of recombinants determined as in Table I (Panel A).
Figure 10 provides a map of DNA to be recombined to clone a recombinant DNA pRP406-SFV using the method of the invention.
Figure 11 shows the results of *in vitro* recombination of the linear DNA shown in Figure 10 (shown on 0.8 % agarose gel). After the joining reaction, I ul of reaction solution was used for transformation. The rest of solution was dcproteinized and electrophoresized.

## Claims

1. A single-step method of joining two linear DNA molecules comprising the step of:
- providing a first linear DNA molecule having a first end and a second end;
- providing a second linear DNA molecule having a first end and a second end;
wherein the first linear DNA molecule has a sequence of nucleotides at either the first end or the second end that is complementary to a sequence of nucleotides at either the first end or the second end of the second linear DNA molecule; and
- incubating the two linear DNA molecules in the presence of a poxvirus DNA polymerase which has 3'-5' exonuclease activity under conditions whereby the two linear DNA molecules are joined.

2. A single-step method of joining three linear DNA molecules comprising:
- providing a first linear DNA molecule having a first end and a second end;
- providing a second linear DNA molecule having a first end and second end;
- providing a third linear DNA molecule having a first end and a second end;
wherein the first linear DNA molecule has a sequence of nucleotides at the first end that is complementary to a sequence of nucleotides at either the first or second end of the second linear DNA molecule and wherein the first linear DNA molecule has a sequence of nucleotides at the second end that is complementary to a sequence of nucleotides at either the first or second end of the third linear DNA molecule, and
- incubating the three linear DNA molecules in the presence of a pox virus DNA polymerase, which has 3'-5' exonuclease activity under conditions whereby the three linear DNA molecules are joined.

3. A single-step method of joining two or more linear DNA molecules according to either of claims 1 or 2 wherein:
- said two or more linear DNA molecules, each have an x and x' strand having opposite polarities, wherein the 5' end of the x' strand of one linear DNA molecule has a sequence of nucleotides that is complementary to the 5' end of the x strand of the linear DNA molecule to which it is to be joined.

4. The method according to any of claims 1-3, wherein the pox virus DNA polymerase is a vaccinia virus DNA polymerase

5. The method according to any one of claims 1 - 4, wherein the two or more linear DNA molecules are incubated in the presence of a DNA polymerase and a single strand DNA binding protein.

6. The method according to claim 5, wherein the single strand DNA binding protein is vaccinia single strand DNA binding protein.

7. The method according to any of claims 1 - 6, wherein the length of the sequence of complementary nucleotides on each linear DNA molecule is between about 5 and about 100 nucleotides.

8. The method according to any one of claims 1 - 6, wherein the length of the sequence of complementary nucleotides on each linear DNA molecule is between about 8 and about 50 nucleotides.

9. The method according to any one of claims 1 - 6,
wherein the length of the sequence of complementary nucleotides on each linear DNA molecule is between about 10 and about 35 nucleotides.

10. A method of circularising linear DNA molecules comprising:
- PCR amplifying a linear DNA molecule in the presence of primers that will introduce substantially complementary nucleic acid sequences onto each end of the linear DNA molecule; and
- incubating in a single-step the PCR amplified linear DNA molecule in the presence of a DNA polymerase which has 3'-5' exonuclease activity under conditions where the linear DNA molecule is circularised;
wherein the DNA polymerase has intrinsic exonuclease activity and circularises the DNA molecule.

11. A method of constructing a recombinant DNA molecule comprising:
- providing a linerized vector DNA molecule;
- providing a PCR amplified linear insert product having ends that are substantially complementary to the ends of the linearized vector DNA molecule to which they are to be joined; and
- incubating in a single-step the linearized vector DNA molecule and PCR amplified linear insert product in the presence of a DNA polymerase under conditions where the two DNA molecules are joined;
wherein the DNA polymerase has intrinsic 3'-5' exonuclease activity and joins the DNA molecules.

12. A method of constructing a recombinant DNA molecule comprising:
- providing a linerized vector DNA molecule
- providing a linear insert DNA molecule having ends that are substantially complementary to the ends of the linearized vector DNA molecule to which they are to be joined; and
- incubating in a single-step the linearized vector DNA molecule and the linear insert DNA molecule in the presence of a pox virus DNA polymerase under conditions where the two DNA molecules are joined;
wherein the pox virus DNA polymerase has intrinsic 3'-5' exonuclease activity and joins the DNA molecules and the linear insert DNA molecule is obtained by linerization of a circular double strand DNA molecule by restricting enzyme digestion, with subsequent attachment of synthetic oligomer linkers onto the ends; or by secondary digestion of an already linear DNA molecule with restriction enzymes, followed by addition of adapter/linkers onto the ends; or by annealing of two appropriately designed synthetic single strand DNA oligomers to form an intact duplex DNA molecule.

13. A method of constructing a recombinant molecule according to claim 11 wherein the linear insert DNA molecule is obtained by:
- providing a first primer DNA molecule having a 5' end that comprises nucleotide sequences that will incorporate sequences that are complementary to the first end of the linearized vector molecule onto the first end of a template DNA molecule and a 3' end that hybridises to a suitable location on the first end of the template DNA molecule;
- providing a second primer DNA molecule having a 5' end that comprises nucleotide sequences that will incorporate nucleotide sequences that are complementary to the second end of the linearized vector molecule onto the second end of the template DNA molecule and a 3' end that hybridises to a suitable locating on the first end of the template DNA molecule; and
- amplifying the template DNA molecule using the polymerase chain reaction with the first and second primers to provide a PCR amplified linear insert product.

14. A method of producing a recombinant DNA product according to claim 13, further comprising:
- transforming the recombinant DNA molecule into a host cell; and
- isolating the recombinant DNA product.

15. The method according to either of claims 13 or 14, wherein the PCR amplified product and the linearized vector DNA molecule are incubated in the presence of a DNA polymerase and a single strand DNA binding protein.

16. The method according to claim 15, wherein the single strand DNA binding protein is vaccinia single strand DNA binding protein.

17. The method according to claim 10, 11 and 13-16, wherein the PCR amplified product and the lineraized vector DNA molecule are incubated in the presence of the DNA polymerase for about 5 to about 60 min.

18. The method according to claims 10, 11 and 13-17, wherein the PCR amplified product and the lineraized vector DNA molecule are incubated in the presence of the DNA polymerase at a temperature of about 35°C to about 40°C.

19. The method according to any one of claims 10 - 18, wherein the sequence of complementary nucleotides on each linear DNA molecule is between about 5 and about 100 nucleotides.

20. The method according to any one of claims 10 - 18, wherein the sequence of complementary nucleotides on each linear DNA molecule is between about 8 and about 50 nucleotides.

21. The method according to any one of claims 10 - 18, wherein the sequence of complementary nucleotide DNA molecule is between about 10 and about 35 nucleotides.

22. The method according to any of claims 10 - 21, wherein the vector is capable of transfecting a prokaryotic or eukaryotic host cell.

23. The method according to claim 22, wherein the vector comprises a vector selected from the group consisting of plasmids, cosmids, phage and BACs.

24. The method according to claim 23, wherein the vector comprises a plasmid.

25. The method according to claim 24, wherein the vector comprises pDW101 (SEQ ID No. 1).

26. A kit for direct cloning of a linear DNA molecule comprising, in separate containers, a DNA polymerase, a single strand DNA binding protein and a reaction buffer, wherein the DNA polymerase has intrinsic 3'-5' exonuclease activity and is capable of performing a DNA joining reaction and further comprising reagents to perform a positive control reaction, wherein the reagents to perform a positive control comprise a linearized vector, an insert DNA with first and second ends having appropriate complementary sequences, a DNA polymerase having intrinsic 3'-5' exonuclease activity that is capable of performing a DNA joining reaction, and reaction buffer.

27. The kit according to claim 26, wherein the reagents to perform a positive control further comprise a single-stranded DNA binding protein.

28. The kit according to claim 27, wherein the linear insert DNA is a PCR-amplified product.

29. The use of vaccinia virus DNA polymerase having exonuclease activity and capable of joining DNA molecules, for cloning DNA molecules.

30. The use of the method according to claims 9 or 12 for site directed mutagenesis.

## Patentansprüche

1. Einzelschrittverfahren zum Verbinden von zwei linearen DNA-Molekülen, umfassend den Schritt:
Bereitstellen eines ersten linearen DNA-Moleküls mit einem ersten Ende und einem zweiten Ende;
Bereitstellen eines zweiten linearen DNA-Moleküls mit einem ersten Ende und einem zweiten Ende;
wobei das erste lineare DNA-Molekül eine Sequenz von Nucleotiden an entweder dem ersten Ende oder dem zweiten Ende hat, die komplementär zu einer Sequenz von Nucleotiden an entweder dem ersten Ende oder dem zweiten Ende des zweiten linearen DNA-Moleküls ist; und
Inkubieren der zwei linearen DNA-Moleküle in Gegenwart einer Pockenvirus-DNA-Polymerase, welche 3'-5'-Exonuclease-Aktivität hat, unter Bedingungen, wodurch die zwei linearen DNA-Moleküle verbunden werden.

2. Einzelschrittverfahren zum Verbinden von drei linearen DNA-Molekülen, umfassend:
Bereitstellen eines ersten linearen DNA-Moleküls mit einem ersten Ende und einem zweiten Ende;
Bereitstellen eines zweiten linearen DNA-Moleküls mit einem ersten Ende und zweiten Ende;
Bereitstellen eines dritten linearen DNA-Moleküls mit einem ersten Ende und einem zweiten Ende;
wobei das erste lineare DNA-Molekül eine Sequenz von Nucleotiden an dem ersten Ende hat, die komplementär zu einer Sequenz von Nucleotiden an entweder dem ersten oder zweiten Ende des zweiten linearen DNA-Moleküls ist und wobei das erste lineare DNA-Molekül eine Sequenz von Nucleotiden an dem zweiten Ende hat, die komplementär zu einer Sequenz von Nucleotiden an entweder dem ersten oder zweiten Ende des dritten linearen DNA-Moleküls ist, und
Inkubieren der drei linearen DNA-Moleküle in Gegenwart einer Pockenvirus-DNA-Polymerase, welche 3'-5'-Exonuclease-Aktivität hat, unter Bedingungen, wodurch die drei linearen DNA-Moleküle verbunden werden.

3. Einzelschrittverfahren zum Verbinden von zwei oder mehr linearen DNA-Molekülen nach entweder Anspruch 1 oder 2, wobei:
die zwei oder mehr linearen DNA-Moleküle jeweils einen x- und x'-Strang mit entgegengesetzten Polaritäten haben, wobei das 5'-Ende des x'-Stranges eines linearen DNA-Moleküls eine Sequenz von Nucleotiden hat, die komplementär zu dem 5'-Ende des x-Stranges des linearen DNA-Moleküls ist, an welches es gebunden werden soll.

4. Verfahren nach einem der Ansprüche 1-3, wobei die Pockenvirus-DNA-Polymerase eine Vacciniavirus-DNA-Polymerase ist.

5. Verfahren nach einem der Ansprüche 1-4, wobei die zwei oder mehr linearen DNA-Moleküle in Gegenwart einer DNA-Polymerase und eines Einzelstrang-DNA bindenden Proteins inkubiert werden.

6. Verfahren nach Anspruch 5, wobei das Einzelstrang-DNA bindende Protein Einzelstrang-DNA bindendes Vaccinia-Protein ist.

7. Verfahren nach einem der Ansprüche 1-6, wobei die Länge der Sequenz von komplementären Nucleotiden auf jedem linearen DNA-Molekül zwischen etwa 5 und etwa 100 Nucleotiden liegt.

8. Verfahren nach einem der Ansprüche 1-6, wobei die Länge der Sequenz von komplementären Nucleotiden auf jedem linearen DNA-Molekül zwischen etwa 8 und etwa 50 Nucleotiden liegt.

9. Verfahren nach einem der Ansprüche 1-6, wobei die Länge der Sequenz von komplementären Nucleotiden auf jedem linearen DNA-Molekül zwischen etwa 10 und etwa 35 Nucleotiden liegt.

10. Verfahren zum Zirkularisieren von linearen DNA-Molekülen, umfassend:
PCR-Amplifizieren eines linearen DNA-Moleküls in Gegenwart von Primern, die im wesentlichen komplementäre Nucleinsäuresequenzen auf jedes Ende des linearen DNA-Moleküls einführen werden; und
Inkubieren in einem einzigen Schritt des PCR-amplifizierten linearen DNA-Moleküls in Gegenwart einer DNA-Polymerase, welche 3'-5'-Exonuclease-Aktivität hat, unter Bedingungen, wo das lineare DNA-Molckül zirkularisiert wird;
wobei die DNA-Polymerase intrinsische Exonuclease-Aktivität hat und das DNA-Molekül zirkularisiert.

11. Verfahren zum Konstruieren eines rekombinanten DNA-Moleküls, umfassend:
Bereitstellen eines linarisierten Vektor-DNA-Moleküls;
Bereitstellen eines PCR-amplifizierten linearen Insert-Produktes mit Enden, die im wesentlichen komplementär zu den Enden des linearisierten Vektor-DNA-Moleküls sind, mit welchen sie verbunden werden sollen; und
Inkubieren in einem einzigen Schritt des linarisierten Vektor-DNA-Moleküls und des PCR-amplifizierten linearen Insert-Produktes in Gegenwart einer DNA-Polymerase unter Bedingungen, wo die zwei DNA-Moleküle verbunden werden;
wobei die DNA-Polymerase intrinsische 3'-5'-Exonuclease-Aktivität hat und die DNA-Moleküle verbindet.

12. Verfahren zum Konstruieren eines rekombinanten DNA-Moleküls, umfassend:
-Bereitstellen eines linearisierten Vektor-DNA-Moleküls
Bereitstellen eines linearen Insert-DNA-Moleküls mit Enden, die im wesentlichen komplementär zu den Enden des linearisierten Vektor-DNA-Moleküls sind, mit welchen sie verbunden werden sollen; und
Inkubieren in einem einzigen Schritt des linearisierten Vektor-DNA-Moleküls und des linearen Insert-DNA-Moleküls in Gegenwart einer Pockenvirus-DNA-Polymerase unter Bedingungen, wo die zwei DNA-Moleküle verbunden werden;
wobei die Pockenvirus-DNA-Polymerase intrinsische 3'-5'-Exonuclease-Aktivität hat und die DNA-Moleküle verbindet und das lineare Insert-DNA-Molekül erhalten wird durch Linearisierung eines zirkulären Doppelstrang-DNA-Moleküls durch Digestion mit Restriktionsenzym mit nachfolgender Anfügung von synthetischen Oligomer-Linkern an die Enden; oder durch sekundäre Digestion eines bereits linearen DNA-Moleküls mit Restriktionsenzymen, gefolgt von Addition von Adaptern/Linkern an die Enden; oder durch Annealen von zwei geeignet gestalteten synthetischen Einzelstrang-DNA-Oligomeren, um ein intaktes Duplex-DNA-Molekül zu erzeugen.

13. Verfahren zum Konstruieren eines rekombinanten Moleküls nach Anspruch 11, wobei das lineare Insert-DNA-Molekül erhalten wird durch:
Bereitstellen eines ersten Primer-DNA-Moleküls mit einem 5'-Ende, das Nucleotidsequenzen umfaßt, die Sequenzen, die komplementär zu dem ersten Ende des linearisierten Vektormoleküls sind, auf das erste Ende eines Matrizen-DNA-Moleküls einbringen werden, und einem 3'-Ende, das an einem geeigneten Standort auf dem ersten Ende des Matrizen-DNA-Moleküls hybridisiert;
Bereitstellen eines zweiten Primer-DNA-Moleküls mit einem 5'-Ende, das Nucleotidsequenzen umfaßt, die Nucleotidsequenzen, die komplementär zu dem zweiten Ende des linearisierten Vektormoleküls sind, auf das zweite Ende des Matrizen-DNA-Moleküls einbringen werden, und einem 3'-Ende, das an einem geeigneten Standort auf dem ersten Ende des Matrizen-DNA-Moleküls hybridisiert; und
Amplifizieren des Matrizen-DNA-Moleküls unter Verwendung der Polymerasekettenreaktion mit dem ersten und zweiten Primer, um ein PCR-amplifiziertes lineares Insertprodukt bereitzustellen.

14. Verfahren zum Erzeugen eines rekombinanten DNA-Produkts nach Anspruch 13, weiterhin umfassend:
Transformieren des rekombinanten DNA-Moleküls in eine Wirtszelle; und
Isolieren des rekombinanten DNA-Produkts.

15. Verfahren nach entweder Anspruch 13 oder 14, wobei das PCR-amplifizierte Produkt und das linearisierte Vektor-DNA-Molekül in Gegenwart einer DNA-Polymerase und eines Einzelstrang-DNA bindenden Proteins inkubiert werden.

16. Verfahren nach Anspruch 15, wobei das Einzelstrang-DNA bindende Protein Einzelstrang-DNA bindendes Vaccinia-Protein ist.

17. Verfahren nach Anspruch 10, 11 und 13-16, wobei das PCR-amplifizierte Produkt und das linearisierte Vektor-DNA-Molekül in Gegenwart der DNA-Polymerase für etwa 5 bis etwa 60 min inkubiert werden.

18. Verfahren nach Anspruch 10, 11 und 13-17, wobei das PCR-amplifizierte Produkt und das linearisierte Vektor-DNA-Molekül in Gegenwart der DNA-Polynterase bei einer Temperatur von etwa 35°C bis etwa 40°C inkubiert werden.

19. Verfahren nach einem der Ansprüche 10-18, wobei die Sequenz von komplementären Nucleotiden auf jedem linearen DNA-Molekül zwischen etwa 5 und etwa 100 Nucleotide ausmacht.

20. Verfahren nach einem der Ansprüche 10-18, wobei die Sequenz von komplementären Nucleotiden auf jedem linearen DNA-Molekül zwischen etwa 8 und etwa 50 Nucleotide ausmacht.

21. Verfahren nach einem der Ansprüche 10-18, wobei die Sequenz von komplementärem Nucleotid des DNA-Moleküls zwischen etwa 10 und etwa 35 Nucleotide ausmacht.

22. Verfahren nach einem der Ansprüche 10-21, wobei der Vektor fähig ist, eine prokaryotische oder eukaryotische Wirtszelle zu transfizieren.

23. Verfahren nach Anspruch 22, wobei der Vektor einen Vektor umfaßt, ausgewählt aus der Gruppe, bestehend aus Plasmiden, Cosmiden, Phage und BACs.

24. Verfahren nach Anspruch 23, wobei der Vektor ein Plasmid umfaßt.

25. Verfahren nach Anspruch 24, wobei der Vektor pDW101 (SEQ ID No. 1) umfaßt.

26. Kit zum direkten Klonen eines linearen DNA-Molcküls, umfassend in gesonderten Behältern eine DNA-Polymerase, ein Einzelstrang-DNA bindendes Protein und einen Reaktionspuffer, wobei die DNA-Polymerase intrinsische 3'-5'-Exonuclease-Aktivität hat und fähig ist, eine DNA verbindende Reaktion durchzuführen, und weiterhin umfassend Reagenzien, um eine positive Kontrollreaktion durchzuführen, wobei die Reagenzien, um eine positive Kontrolle durchzuführen, einen linearisierten Vektor, eine Insert-DNA mit erstem und zweitem Ende mit geeigneten komplementären Sequenzen, eine DNA-Polymerase mit intrinsischer 3'-5'-Exonuclease-Aktivität, die zum Durchführen einer DNA verbindenden Reaktion fähig ist, und Reaktionspuffer umfassen.

27. Kit nach Anspruch 26, wobei die Reagenzien, um eine positive Kontrolle durchzuführen, weiterhin ein Einzelstrang-DNA bindendes Protein umfassen.

28. Kit nach Anspruch 27, wobei die lineare Insert-DNA ein PCR-amplifiziertes Produkt ist.

29. Verwendung von Vacciniavirus-DNA-Polymerase mit Exonuclease-Aktivität und fähig zum Verbinden von DNA-Molekülen zum Klonen von DNA-Molekülen.

30. Verwendung des Verfahrens nach den Ansprüchen 9 oder 12 für stellengerichtete Mutagenese.

## Revendications

1. Procédé en une seule étape d'assemblage de deux molécules d'ADN linéaire comprenant l'étape consistant à :
fournir une première molécule d'ADN linéaire ayant une première extrémité et une deuxième extrémité;
fournir une deuxième molécule d'ADN linéaire ayant une première extrémité et une deuxième extrémité;
où la première molécule d'ADN linéaire a une séquence de nucléotides à la premier extrémité ou à la deuxième extrémité, qui est complémentaire à une séquence de nucléotides à la première extrémité ou à la deuxième extrémité de la deuxième molécule d'ADN linéaire; et
incuber les deux molécules d'ADN linéaire en présence d'ADN polymérase de pox virus possédant une activité cxonucléase 3' - 5' dans des conditions dans lesquelles les deux molécules d'ADN linéaire sont assemblées.

2. Procédé en une seule étape d'assemblage de trois molécules d'ADN linéaire comprenant :
fournir une première molécule d'ADN linéaire ayant une première extrémité et une deuxième extrémité;
fournir une deuxième molécule d'ADN linéaire ayant une première extrémité et une deuxième extrémité;
fournir une troisième molécule d'ADN linéaire ayant une première extrémité et une deuxième extrémité;
où la première molécule d'ADN linéaire a une séquence de nucléotides à la premier extrémité qui est complémentaire à une séquence de nucléotides à la première extrémité ou à la deuxième extrémité de la deuxième molécule d'ADN linéaire et où la première molécule d'ADN linéaire a une séquence de nucléotides à la deuxième extrémité qui est complémentaire à une séquence de nucléotides à la première ou à la deuxième extrémité de la troisième molécule d'ADN linéaire; et
incuber les trois molécules d'ADN linéaire en présence d'ADN polymérase de pox virus possédant une activité exonucléase 3' - 5' dans des conditions dans lesquelles les trois molécules d'ADN linéaire sont assemblées.

3. Procédé en une seule étape d'assemblage de deux molécules ou plus d'ADN linéaire selon la revendication 1 ou 2, où :
lesdites deux molécules ou plus d'ADN linéaire, ont chacune un brin x et x' ayant des polarités opposées, où l'extrémité 5' du brin x' d'une molécule d'ADN linéaire a une séquence de nucléotides qui est complémentaire à l'extrémité 5' du brin x de la molécule d'ADN linéaire à laquelle elle doit être jointe.

4. Le procédé selon l'une quelconque des revendications 1 - 3, dans lequel l'ADN polymérase de pox virus est une ADN polymérase du virus de la vaccine.

5. Le procédé selon l'une quelconque des revendications 1 - 4, dans lequel les deux molécules ou plus d'ADN linéaire sont incubées en présence d'une ADN polymérase et d'une protéine de liaison à un ADN simple brin.

6. Le procédé selon la revendication 5, dans lequel la protéine de liaison à un ADN simple brin est une protéine de liaison à un ADN simple brin de vaccine.

7. Le procédé selon l'une quelconque des revendications 1 - 6, dans lequel la longueur de la séquence de nucléotides complémentaires sur chaque molécule d'ADN linéaire, fait d'entre environ 5 et environ 100 nucléotides.

8. Le procédé selon l'une quelconque des revendications 1 - 6, dans lequel la longueur de la séquence de nucléotides complémentaires sur chaque molécule d'ADN linéaire, fait d'entre environ 8 et 50 environ nucléotides.

9. Le procédé selon l'une quelconque des revendications 1 - 6, dans lequel la longueur de la séquence de nucléotides complémentaires sur chaque molécule d'ADN linéaire, fait d'entre environ 10 et 35 environ nucléotides.

10. Procédé de circularisation de molécules d'ADN linéaire, comprenant :
amplifier par PCR une molécule d'ADN linéaire en présence d'amorces qui introduiront des séquences d'acides nucléiques sensiblement complémentaires sur chaque extrémité de la molécule d'ADN linéaire; et
incuber en une seule étape la molécule d'ADN linéaire amplifiée par PCR en présence d'une ADN polymérase qui a une activité exonucléase 3' - 5' dans des conditions dans lesquelles la molécule d'ADN linéaire est circularisée;
où l'ADN polymérase a une activité exonucléase intrinsèque et circularise la molécule d'ADN.

11. Procédé de construction d'une molécule d'ADN recombinant, comprenant :
fournir une molécule d'ADN de vecteur linéarisé;
fournir un produit d'insertion linéaire amplifié par PCR ayant des extrémités qui sont sensiblement complémentaires aux extrémités de la molécule d'ADN de vecteur linéarisé auxquelles elles doivent être jointes; et
incuber en une seule étape la molécule d'ADN de vecteur linéarisé et le produit d'insertion linéaire amplifié par PCR en présence d'une ADN polymérase dans des conditions dans lesquelles les deux molécules d'ADN sont assemblées;
où l'ADN polymérase a une activité exonucléase 3' - 5' intrinsèque et joint les molécules d'ADN.

12. Procédé de construction d'une molécule d'ADN recombinant, comprenant :
fournir une molécule d'ADN de vecteur linéarisé
fournir une molécule d'ADN d'insertion linéaire ayant des extrémités qui sont sensiblement complémentaires aux extrémités de la molécule d'ADN de vecteur linéarisé auxquelles elles doivent être jointes; et
incuber en une seule étape la molécule d'ADN de vecteur linéarisé et la molécule d'ADN d'insertion linéaire en présence d'une ADN polymérase de pox virus dans des conditions dans lesquelles les deux molécules d'ADN sont assemblées;
où l'ADN polymérase de pox virus a une activité exonucléase 3' - 5' intrinsèque et joint les molécules d'ADN, et la molécule d'ADN d'insertion linéaire est obtenue par la linéarisation d'une molécule d'ADN double brin circulaire par la digestion avec une enzyme de restriction, avec la fxation subséquente de lieurs oligomères synthétiques aux extrémités; ou par la digestion secondaire d'une molécule d'ADN déjà linéaire avec des enzymes de restriction, suivie par l'addition d'adaptateurs/lieurs aux extrémités; ou par l'hybridation de deux oligomères d'ADN simple brin synthétiques, conçus de manière appropriée, pour former une molécule d'ADN duplex intacte.

13. Procédé de construction d'une molécule recombinante selon la revendication 11, dans lequel la molécule d'ADN d'insertion linéaire est obtenue en :
fournissant une première molécule d'ADN d'amorce linéaire ayant une extrémité 5' qui comprend des séquences de nucléotides qui incorporeront des séquences qui sont complémentaires à la première extrémité de la molécule de vecteur linéarisée, sur la première extrémité d'une molécule d'ADN de matrice, et une extrémité 3' qui s'hybride à un emplacement approprié sur la première extrémité de la molécule d'ADN de matrice;
fournissant une deuxième molécule d'ADN d'amorce ayant une extrémité 5' qui comprend des séquences de nucléotides qui incorporeront des séquences de nucléotides qui sont complémentaires à la deuxième extrémité de la molécule de vecteur linéarisée sur la deuxième extrémité de la molécule d'ADN de matrice, et une extrémité 3' qui s'hybride à un emplacement approprié sur la première extrémité de la molécule d'ADN de matrice; et
amplifiant la molécule d'ADN de matrice par réaction de polymérase en chaîne avec les première et deuxième amorces pour donner un produit d'insertion linéaire amplifié par PCR.

14. Procédé de production d'un produit d'ADN recombinant selon la revendication 13, comprenant en outre :
transformer la molécule d'ADN recombinant dans une cellule hôte; et
isoler le produit d'ADN recombinant.

15. Le procédé selon la revendication 13 ou 14, dans lequel le produit amplifié par PCR et la molécule d'ADN de vecteur linéarisé, sont incubés en présence d'une ADN polymérase et d'une protéine de liaison à un ADN simple brin.

16. Le procédé selon la revendication 15, dans lequel la protéine de liaison à un ADN simple brin est une protéine de liaison à un ADN simple brin de vaccine.

17. Le procédé selon les revendications 10, 11 et 13 - 16, dans lequel le produit amplifié par PCR et la molécule d'ADN de vecteur linéarisé, sont incubés en présence de l'ADN polymérase pendant environ 5 à environ 60 minutes.

18. Le procédé selon les revendications 10, 11 et 13 - 17, dans lequel le produit amplifié par PCR et la molécule d'ADN de vecteur linéarisé, sont incubés en présence de l'ADN polymérase à une température d'environ 35°C à environ 40°C.

19. Le procédé selon l'une quelconque des revendications 10 - 18, dans lequel la séquence de nucléotides complémentaires sur chaque molécule d'ADN linéaire, fait d'entre environ 5 et environ 100 nucléotides.

20. Le procédé selon l'une quelconque des revendications 10 - 18, dans lequel la séquence de nucléotides complémentaires sur chaque molécule d'ADN linéaire, fait d'entre environ 8 et environ 50 nucléotides.

21. Le procédé selon l'une quelconque des revendications 10 - 18, dans lequel la séquence de nucléotides complémentaires sur la molécule d'ADN linéaire, fait d'entre environ 10 et environ 35 nucléotides.

22. Le procédé selon l'une quelconque des revendications 10 - 21, dans lequel le vecteur est capable de transfecter une cellule hôte procaryote ou eucaryote.

23. Le procédé selon la revendication 22, dans lequel le vecteur comprend un vecteur sélectionné parmi le groupe consistant en plasmides, cosmides, phage et BAC.

24. Le procédé selon la revendication 23, dans lequel le vecteur comprend un plasmide.

25. Le procédé selon la revendication 24, dans lequel le vecteur comprend pDW101 (SEQ ID NO:1).

26. Kit de clonage direct d'une molécule d'ADN linéaire, comprenant, dans des récipients séparés, une ADN polymérase, une protéine de liaison à un ADN simple brin et un tampon de réaction, où l'ADN polymérase a une activité exonucléase 3' - 5' intrinsèque et est capable d'effectuer une réaction d'assemblage d'ADN, et comprend en outre des réactifs pour effectuer une réaction de contrôle positif, où les réactifs pour effectuer une réaction de contrôle positif comprennent un vecteur linéarisé, un ADN d'insertion avec des première et deuxième extrémités ayant des séquences complémentaires appropriées, une ADN polymérase ayant une activité exonucléase 3' - 5' intrinsèque qui est capable d'effectuer une réaction d'assemblage d'ADN, et un tampon de réaction.

27. Le kit selon la revendication 26, dans lequel les réactifs pour effectuer un contrôle positif comprennent en outre une protéine de liaison à un ADN simple brin.

28. Le kit selon la revendication 27, dans lequel l'ADN d'insertion linéaire est un produit amplifié par PCR.

29. Utilisation d'ADN polymérase du virus de la vaccine ayant une activité exonucléase et capable d'assembler des molécules d'ADN, dans le clonage de molécules d'ADN.

30. Utilisation du procédé selon la revendication 9 ou 12, dans la mutagenèse dirigée sur site.


### SEQUENCE LISTING

<110> University of Guelph
   Evans, David H.
   Willer, David O.
   Yao, Xiao-Dan
<120> DNA JOINING METHOD
<130> 6580-241
<150> US 60/187,401
   <151> 2000-03-07
<150> US
   <151> 2001-01-25
<150> CA 2,317,865
   <151> 2000-08-01
<160> 11
<170> PatentIn version 3.0
<210> 1
   <211> 2958
   <212> DNA
   <213> Artificial
<220>
   <223> pDW101
<400> 1
   ctaaattgta agcgttaata ttttgttaaa attcgcgtta aatttttgtt aaatcagctc     60
   attttttaac caataggccg aaatcggcaa aatcccttat aaatcaaaag aatagaccga    120
   gatagggttg agtgttgttc cagtttggaa caagagtcca ctattaaaga acgtggactc    180
   caacgtcaaa gggcgaaaaa ccgtctatca gggcgatggc ccactacgtg aaccatcacc    240
   ctaatcaagt tttttggggt cgaggtgccg taaagcacta aatcggaacc ctaaagggag    300
   cccccgattt agagcttgac ggggaaagcc ggcgaacgtg gcgagaaagg aagggaagaa    360
   agcgaaagga gcgggcgcta gggcgctggc aagtgtagcg gtcacgctgc gcgtaaccac    420
   cacacccgcc gcgcttaatg cgccgctaca gggcgcgtcc cattcgccat tcaggctgcg    480
   caactgttgg gaagggcgat cggtgcgggc ctcttcgcta ttacgccagc tggcgaaagg    540
   gggatgtgct gcaaggcgat taagttgggt aacgccaggg ttttcccagt cacgacgttg    600
   taaaacgacg gccagtgaat tgtaatacga ctcactatag ggcgaattgg agctccaccg    660
   cggtggcggc cgctctagaa ctagtggatc ccccgggctg caggaattcg atatcaagct    720
   tatcgatacc gtcgacctcg agggggggcc cggtacccag cttttgttcc ctttagtgag    780
   ggttaattgc gcgcttggcg taatcatggt catagctgtt tcctgtgtga aattgttatc    840
   cgctcacaat tccacacaac atacgagccg gaagcataaa gtgtaaagcc tggggtgcct    900
   aatgagtgag ctaactcaca ttaattgcgt tgcgctcact gcccgctttc cagtcgggaa    960
   acctgtcgtg ccagctgcat taatgaatcg gccaacgcgc ggggagaggc ggtttgcgta   1020
   ttgggcgctc ttccgcttcc tcgctcactg actcgctgcg ctcggtcgtt cggctgcggc   1080
   gagcggtatc agctcactca aaggcggtaa tacggttatc cacagaatca ggggataacg   1140
   caggaaagaa catgtgagca aaaggccagc aaaaggccag gaaccgtaaa aaggccgcgt   1200
   tgctggcgtt tttccatagg ctccgccccc ctgacgagca tcacaaaaat cgacgctcaa   1260
   gtcagaggtg gcgaaacccg acaggactat aaagatacca ggcgtttccc cctggaagct   1320
   ccctcgtgcg ctctcctgtt ccgaccctgc cgcttaccgg atacctgtcc gcctttctcc   1380
   cttcgggaag cgtggcgctt tctcatagct cacgctgtag gtatctcagt tcggtgtagg   1440
   tcgttcgctc caagctgggc tgtgtgcacg aaccccccgt tcagcccgac cgctgcgcct   1500
   tatccggtaa ctatcgtctt gagtccaacc cggtaagaca cgacttatcg ccactggcag   1560
   cagccactgg taacaggatt agcagagcga ggtatgtagg cggtgctaca gagttcttga   1620
   agtggtggcc taactacggc tacactagaa ggacagtatt tggtatctgc gctctgctga   1680
   agccagttac cttcggaaaa agagttggta gctcttgatc cggcaaacaa accaccgctg   1740
   gtagcggtgg tttttttgtt tgcaagcagc agattacgcg cagaaaaaaa ggatctcaag   1800
   aagatccttt gatcttttct acggggtctg acgctcagtg gaacgaaaac tcacgttaag   1860
   ggattttggt catgagatta tcaaaaagga tcttcaccta gatcctttta aattaaaaat   1920
   gaagttttaa atcaatctaa agtatatatg agtaaacttg gtctgacagt taccaatgct   1980
   taatcagtga ggcacctatc tcagcgatct gtctatttcg ttcatccata gttgcctgac   2040
   tccccgtcgt gtagataact acgatacggg agggcttacc atctggcccc agtgctgcaa   2100
   tgataccgcg agacccacgc tcaccggctc cagatttatc agcaataaac cagccagccg   2160
   gaagggccga gcgcagaagt ggtcctgcaa ctttatccgc ctccatccag tctattaatt   2220
   gttgccggga agctagagta agtagttcgc cagttaatag tttgcgcaac gttgttgcca   2280
   ttgctacagg catcgtggtg tcacgctcgt cgtttggtat ggcttcattc agctccggtt   2340
   cccaacgatc aaggcgagtt acatgatccc ccatgttgtg caaaaaagcg gttagctcct   2400
   tcggtcctcc gatcgttgtc agaagtaagt tggccgcagt gttatcactc atggttatgg   2460
   cagcactgca taattctctt actgtcatgc catccgtaag atgcttttct gtgactggtg   2520
   agtactcaac caagtcattc tgagaatagt gtatgcggcg accgagttgc tcttgcccgg   2580
   cgtcaatacg ggataatacc gcgccacata gcagaacttt aaaagtgctc atcattggaa   2640
   aacgttcttc ggggcgaaaa ctctcaagga tcttaccgct gttgagatcc agttcgatgt 2700
   aacccactcg tgcacccaac tgatcttcag catcttttac tttcaccagc gtttctgggt 2760
   gagcaaaaac aggaaggcaa aatgccgcaa aaaagggaat aagggcgaca cggaaatgtt 2820
   gaatactcat actcttcctt tttcaatatt attgaagcat ttatcagggt tattgtctca 2880
   tgagcggata catatttgaa tgtatttaga aaaataaaca aataggggtt ccgcgcacat 2940
   ttccccgaaa agtgccac 2958
<210> 2
   <211> 2958
   <212> DNA
   <213> Artificial
<220>
   <223> pDW101 cut with EcoRI
<400> 2
   aattcgatat caagcttatc gataccgtcg acctcgaggg ggggcccggt acccagcttt     60
   tgttcccttt agtgagggtt aattgcgcgc ttggcgtaat catggtcata gctgtttcct    120
   gtgtgaaatt gttatccgct cacaattcca cacaacatac gagccggaag cataaagtgt    180
   aaagcctggg gtgcctaatg agtgagctaa ctcacattaa ttgcgttgcg ctcactgccc    240
   gctttccagt cgggaaacct gtcgtgccag ctgcattaat gaatcggcca acgcgcgggg    300
   agaggcggtt tgcgtattgg gcgctcttcc gcttcctcgc tcactgactc gctgcgctcg    360
   gtcgttcggc tgcggcgagc ggtatcagct cactcaaagg cggtaatacg gttatccaca    420
   gaatcagggg ataacgcagg aaagaacatg tgagcaaaag gccagcaaaa ggccaggaac    480
   cgtaaaaagg ccgcgttgct ggcgtttttc cataggctcc gcccccctga cgagcatcac    540
   aaaaatcgac gctcaagtca gaggtggcga aacccgacag gactataaag ataccaggcg    600
   tttccccctg gaagctccct cgtgcgctct cctgttccga ccctgccgct taccggatac    660
   ctgtccgcct ttctcccttc gggaagcgtg gcgctttctc atagctcacg ctgtaggtat    720
   ctcagttcgg tgtaggtcgt tcgctccaag ctgggctgtg tgcacgaacc ccccgttcag    780
   cccgaccgct gcgccttatc cggtaactat cgtcttgagt ccaacccggt aagacacgac    840
   ttatcgccac tggcagcagc cactggtaac aggattagca gagcgaggta tgtaggcggt    900
   gctacagagt tcttgaagtg gtggcctaac tacggctaca ctagaaggac agtatttggt    960
   atctgcgctc tgctgaagcc agttaccttc ggaaaaagag ttggtagctc ttgatccggc   1020
   aaacaaacca ccgctggtag cggtggtttt tttgtttgca agcagcagat tacgcgcaga   1080
   aaaaaaggat ctcaagaaga tcctttgatc ttttctacgg ggtctgacgc tcagtggaac   1140
   gaaaactcac gttaagggat tttggtcatg agattatcaa aaaggatctt cacctagatc   1200
   cttttaaatt aaaaatgaag ttttaaatca atctaaagta tatatgagta aacttggtct   1260
   gacagttacc aatgcttaat cagtgaggca cctatctcag cgatctgtct atttcgttca   1320
   tccatagttg cctgactccc cgtcgtgtag ataactacga tacgggaggg cttaccatct   1380
   ggccccagtg ctgcaatgat accgcgagac ccacgctcac cggctccaga tttatcagca   1440
   ataaaccagc cagccggaag ggccgagcgc agaagtggtc ctgcaacttt atccgcctcc   1500
   atccagtcta ttaattgttg ccgggaagct agagtaagta gttcgccagt taatagtttg   1560
   cgcaacgttg ttgccattgc tacaggcatc gtggtgtcac gctcgtcgtt tggtatggct   1620
   tcattcagct ccggttccca acgatcaagg cgagttacat gatcccccat gttgtgcaaa   1680
   aaagcggtta gctccttcgg tcctccgatc gttgtcagaa gtaagttggc cgcagtgtta   1740
   tcactcatgg ttatggcagc actgcataat tctcttactg tcatgccatc cgtaagatgc   1800
   ttttctgtga ctggtgagta ctcaaccaag tcattctgag aatagtgtat gcggcgaccg   1860
   agttgctctt gcccggcgtc aatacgggat aataccgcgc cacatagcag aactttaaaa   1920
   gtgctcatca ttggaaaacg ttcttcgggg cgaaaactct caaggatctt accgctgttg   1980
   agatccagtt cgatgtaacc cactcgtgca cccaactgat cttcagcatc ttttactttc   2040
   accagcgttt ctgggtgagc aaaaacagga aggcaaaatg ccgcaaaaaa gggaataagg   2100
   gcgacacgga aatgttgaat actcatactc ttcctttttc aatattattg aagcatttat   2160
   cagggttatt gtctcatgag cggatacata tttgaatgta tttagaaaaa taaacaaata   2220
   ggggttccgc gcacatttcc ccgaaaagtg ccacctaaat tgtaagcgtt aatattttgt   2280
   taaaattcgc gttaaatttt tgttaaatca gctcattttt taaccaatag gccgaaatcg   2340
   gcaaaatccc ttataaatca aaagaataga ccgagatagg gttgagtgtt gttccagttt   2400
   ggaacaagag tccactatta aagaacgtgg actccaacgt caaagggcga aaaaccgtct   2460
   atcagggcga tggcccacta cgtgaaccat caccctaatc aagttttttg gggtcgaggt   2520
   gccgtaaagc actaaatcgg aaccctaaag ggagcccccg atttagagct tgacggggaa   2580
   agccggcgaa cgtggcgaga aaggaaggga agaaagcgaa aggagcgggc gctagggcgc   2640
   tggcaagtgt agcggtcacg ctgcgcgtaa ccaccacacc cgccgcgctt aatgcgccgc   2700
   tacagggcgc gtcccattcg ccattcaggc tgcgcaactg ttgggaaggg cgatcggtgc   2760
   gggcctcttc gctattacgc cagctggcga aagggggatg tgctgcaagg cgattaagtt   2820
   gggtaacgcc agggttttcc cagtcacgac gttgtaaaac gacggccagt gaattgtaat   2880
   acgactcact atagggcgaa ttggagctcc accgcggtgg cggccgctct agaactagtg   2940
   gatcccccgg gctgcagg                                                 2958
<210> 3
   <211> 2958
   <212> DNA
   <213> Artificial
<220>
   <223> pDW101 cut with XhoI
<400> 3
   tcgagggggg gcccggtacc cagcttttgt tccctttagt gagggttaat tgcgcgcttg 60
   gcgtaatcat ggtcatagct gtttcctgtg tgaaattgtt atccgctcac aattccacac 120
   aacatacgag ccggaagcat aaagtgtaaa gcctggggtg cctaatgagt gagctaactc 180
   acattaattg cgttgcgctc actgcccgct ttccagtcgg gaaacctgtc gtgccagctg 240
   cattaatgaa tcggccaacg cgcggggaga ggcggtttgc gtattgggcg ctcttccgct 300
   tcctcgctca ctgactcgct gcgctcggtc gttcggctgc ggcgagcggt atcagctcac 360
   tcaaaggcgg taatacggtt atccacagaa tcaggggata acgcaggaaa gaacatgtga 420
   gcaaaaggcc agcaaaaggc caggaaccgt aaaaaggccg cgttgctggc gtttttccat 480
   aggctccgcc cccctgacga gcatcacaaa aatcgacgct caagtcagag gtggcgaaac 540
   ccgacaggac tataaagata ccaggcgttt ccccctggaa gctccctcgt gcgctctcct 600
   gttccgaccc tgccgcttac cggatacctg tccgcctttc tcccttcggg aagcgtggcg 660
   ctttctcata gctcacgctg taggtatctc agttcggtgt aggtcgttcg ctccaagctg 720
   ggctgtgtgc acgaaccccc cgttcagccc gaccgctgcg ccttatccgg taactatcgt 780
   cttgagtcca acccggtaag acacgactta tcgccactgg cagcagccac tggtaacagg 840
   attagcagag cgaggtatgt aggcggtgct acagagttct tgaagtggtg gcctaactac 900
   ggctacacta gaaggacagt atttggtatc tgcgctctgc tgaagccagt taccttcgga 960
   aaaagagttg gtagctcttg atccggcaaa caaaccaccg ctggtagcgg tggttttttt 1020
   gtttgcaagc agcagattac gcgcagaaaa aaaggatctc aagaagatcc tttgatcttt 1080
   tctacggggt ctgacgctca gtggaacgaa aactcacgtt aagggatttt ggtcatgaga 1140
   ttatcaaaaa ggatcttcac ctagatcctt ttaaattaaa aatgaagttt taaatcaatc 1200
   taaagtatat atgagtaaac ttggtctgac agttaccaat gcttaatcag tgaggcacct 1260
   atctcagcga tctgtctatt tcgttcatcc atagttgcct gactccccgt cgtgtagata 1320
   actacgatac gggagggctt accatctggc cccagtgctg caatgatacc gcgagaccca 1380
   cgctcaccgg ctccagattt atcagcaata aaccagccag ccggaagggc cgagcgcaga 1440
   agtggtcctg caactttatc cgcctccatc cagtctatta attgttgccg ggaagctaga 1500
   gtaagtagtt cgccagttaa tagtttgcgc aacgttgttg ccattgctac aggcatcgtg 1560
   gtgtcacgct cgtcgtttgg tatggcttca ttcagctccg gttcccaacg atcaaggcga 1620
   gttacatgat cccccatgtt gtgcaaaaaa gcggttagct ccttcggtcc tccgatcgtt 1680
   gtcagaagta agttggccgc agtgttatca ctcatggtta tggcagcact gcataattct 1740
   cttactgtca tgccatccgt aagatgcttt tctgtgactg gtgagtactc aaccaagtca 1800
   ttctgagaat agtgtatgcg gcgaccgagt tgctcttgcc cggcgtcaat acgggataat 1860
   accgcgccac atagcagaac tttaaaagtg ctcatcattg gaaaacgttc ttcggggcga 1920
   aaactctcaa ggatcttacc gctgttgaga tccagttcga tgtaacccac tcgtgcaccc 1980
   aactgatctt cagcatcttt tactttcacc agcgtttctg ggtgagcaaa aacaggaagg 2040
   caaaatgccg caaaaaaggg aataagggcg acacggaaat gttgaatact catactcttc 2100
   ctttttcaat attattgaag catttatcag ggttattgtc tcatgagcgg atacatattt 2160
   gaatgtattt agaaaaataa acaaataggg gttccgcgca catttccccg aaaagtgcca 2220
   cctaaattgt aagcgttaat attttgttaa aattcgcgtt aaatttttgt taaatcagct 2280
   cattttttaa ccaataggcc gaaatcggca aaatccctta taaatcaaaa gaatagaccg 2340
   agatagggtt gagtgttgtt ccagtttgga acaagagtcc actattaaag aacgtggact 2400
   ccaacgtcaa agggcgaaaa accgtctatc agggcgatgg cccactacgt gaaccatcac 2460
   cctaatcaag ttttttgggg tcgaggtgcc gtaaagcact aaatcggaac cctaaaggga 2520
   gcccccgatt tagagcttga cggggaaagc cggcgaacgt ggcgagaaag gaagggaaga 2580
   aagcgaaagg agcgggcgct agggcgctgg caagtgtagc ggtcacgctg cgcgtaacca 2640
   ccacacccgc cgcgcttaat gcgccgctac agggcgcgtc ccattcgcca ttcaggctgc 2700
   gcaactgttg ggaagggcga tcggtgcggg cctcttcgct attacgccag ctggcgaaag 2760
   ggggatgtgc tgcaaggcga ttaagttggg taacgccagg gttttcccag tcacgacgtt 2820
   gtaaaacgac ggccagtgaa ttgtaatacg actcactata gggcgaattg gagctccacc 2880
   gcggtggcgg ccgctctaga actagtggat cccccgggct gcaggaattc gatatcaagc 2940
   ttatcgatac cgtcgacc 2958
<210> 4
   <211> 2958
   <212> DNA
   <213> Artificial
<220>
   <223> pDW101 cut with HindIII
<400> 4
   agcttatcga taccgtcgac ctcgaggggg ggcccggtac ccagcttttg ttccctttag      60
   tgagggttaa ttgcgcgctt ggcgtaatca tggtcatagc tgtttcctgt gtgaaattgt     120
   tatccgctca caattccaca caacatacga gccggaagca taaagtgtaa agcctggggt     180
   gcctaatgag tgagctaact cacattaatt gcgttgcgct cactgcccgc tttccagtcg     240
   ggaaacctgt cgtgccagct gcattaatga atcggccaac gcgcggggag aggcggtttg     300
   cgtattgggc gctcttccgc ttcctcgctc actgactcgc tgcgctcggt cgttcggctg     360
   cggcgagcgg tatcagctca ctcaaaggcg gtaatacggt tatccacaga atcaggggat     420
   aacgcaggaa agaacatgtg agcaaaaggc cagcaaaagg ccaggaaccg taaaaaggcc     480
   gcgttgctgg cgtttttcca taggctccgc ccccctgacg agcatcacaa aaatcgacgc     540
   tcaagtcaga ggtggcgaaa cccgacagga ctataaagat accaggcgtt tccccctgga     600
   agctccctcg tgcgctctcc tgttccgacc ctgccgctta ccggatacct gtccgccttt     660
   ctcccttcgg gaagcgtggc gctttctcat agctcacgct gtaggtatct cagttcggtg     720
   taggtcgttc gctccaagct gggctgtgtg cacgaacccc ccgttcagcc cgaccgctgc     780
   gccttatccg gtaactatcg tcttgagtcc aacccggtaa gacacgactt atcgccactg     840
   gcagcagcca ctggtaacag gattagcaga gcgaggtatg taggcggtgc tacagagttc     900
   ttgaagtggt ggcctaacta cggctacact agaaggacag tatttggtat ctgcgctctg     960
   ctgaagccag ttaccttcgg aaaaagagtt ggtagctctt gatccggcaa acaaaccacc    1020
   gctggtagcg gtggtttttt tgtttgcaag cagcagatta cgcgcagaaa aaaggatctc    1080
   caagaagatc ctttgatctt ttctacgggg tctgacgctc agtggaacga aaactcacgt    1140
   taagggattt tggtcatgag attatcaaaa aggatcttca cctagatcct tttaaattaa    1200
   aaatgaagtt ttaaatcaat ctaaagtata tatgagtaaa cttggtctga cagttaccaa 1260
   tgcttaatca gtgaggcacc tatctcagcg atctgtctat ttcgttcatc catagttgcc 1320
   tgactccccg tcgtgtagat aactacgata cgggagggct taccatctgg ccccagtgct 1380
   gcaatgatac cgcgagaccc acgctcaccg gctccagatt tatcagcaat aaaccagcca 1440
   gccggaaggg ccgagcgcag aagtggtcct gcaactttat ccgcctccat ccagtctatt 1500
   aattgttgcc gggaagctag agtaagtagt tcgccagtta atagtttgcg caacgttgtt 1560
   gccattgcta caggcatcgt ggtgtcacgc tcgtcgtttg gtatggcttc attcagctcc 1620
   ggttcccaac gatcaaggcg agttacatga tcccccatgt tgtgcaaaaa agcggttagc 1680
   tccttcggtc ctccgatcgt tgtcagaagt aagttggccg cagtgttatc actcatggtt 1740
   atggcagcac tgcataattc tcttactgtc atgccatccg taagatgctt ttctgtgact 1800
   ggtgagtact caaccaagtc attctgagaa tagtgtatgc ggcgaccgag ttgctcttgc 1860
   ccggcgtcaa tacgggataa taccgcgcca catagcagaa ctttaaaagt gctcatcatt 1920
   ggaaaacgtt cttcggggcg aaaactctca aggatcttac cgctgttgag atccagttcg 1980
   atgtaaccca ctcgtgcacc caactgatct tcagcatctt ttactttcac cagcgtttct 2040
   gggtgagcaa aaacaggaag gcaaaatgcc gcaaaaaagg gaataagggc gacacggaaa 2100
   tgttgaatac tcatactctt cctttttcaa tattattgaa gcatttatca gggttattgt 2160
   ctcatgagcg gatacatatt tgaatgtatt tagaaaaata aacaaatagg ggttccgcgc 2220
   acatttcccc gaaaagtgcc acctaaattg taagcgttaa tattttgtta aaattcgcgt 2280
   taaatttttg ttaaatcagc tcatttttta accaataggc cgaaatcggc aaaatccctt 2340
   ataaatcaaa agaatagacc gagatagggt tgagtgttgt tccagtttgg aacaagagtc 2400
   cactattaaa gaacgtggac tccaacgtca aagggcgaaa aaccgtctat cagggcgatg 2460
   gcccactacg tgaaccatca ccctaatcaa gttttttggg gtcgaggtgc cgtaaagcac 2520
   taaatcggaa ccctaaaggg agcccccgat ttagagcttg acggggaaag ccggcgaacg 2580
   tggcgagaaa ggaagggaag aaagcgaaag gagcgggcgc tagggcgctg gcaagtgtag 2640
   cggtcacgct gcgcgtaacc accacacccg ccgcgcttaa tgcgccgcta cagggcgcgt 2700
   cccattcgcc attcaggctg cgcaactgtt gggaagggcg atcggtgcgg gcctcttcgc 2760
   tattacgcca gctggcgaaa gggggatgtg ctgcaaggcg attaagttgg gtaacgccag 2820
   ggttttccca gtcacgacgt tgtaaaacga cggccagtga attgtaatac gactcactat 2880
   agggcgaatt ggagctccac cgcggtggcg gccgctctag aactagtgga tcccccgggc 2940
   tgcaggaatt cgatatca 2958
<210> 5
   <211> 35
   <212> DNA
   <213> Artificial
<220>
   <223> Primer #1
<400> 5
   ttctagagcg gccagaaaca ggcatcttac gcgtg 35
<210> 6
   <211> 34
   <212> DNA
   <213> Artificial
<220>
   <223> Primer #2
<400> 6
   tccaccgcgg tggcggccac ggaaacgcct tggt 34
<210> 7
   <211> 58
   <212> DNA
   <213> Artificial
<220>
   <223> pDW101 was digested with NOtI
<400> 7
   actagttcta gagcggccgc caccgcggtt gatcaagatc tcgccggcgg tggcgcca 58
<210> 8
   <211> 2502
   <212> DNA
   <213> Artificial
<220>
   <223> pRP406
<400> 8
   tgaccgctac acttgccagc gccctagcgc ccgctccttt cgctttcttc ccttcctttc 60
   tcgccacgtt cgccggcttt ccccgtcaag ctctaaatcg ggggctccct ttagggttcc 120
   gatttagtgc tttacggcac ctcgacccca aaaaacttga ttagggtgat ggttcacgta 180
   gtgggccatc gccctgatag acggtttttc gccctttgac gttggagtcc acgttcttta 240
   atagtggact cttgttccaa actggaacaa cactcaaccc tatctcggtc tattcttttg 300
   atttataagg gattttgccg atttcggcct attggttaaa aaatgagctg atttaacaaa 360
   aatttaacgc gaattttaac aaaatattaa cgcttacaat ttccattcgc cattcaggct 420
   gcgcaactgt tgggaagggc gatcggtgcg ggcctcttcg ctattacgcc agctggcgaa 480
   agggggatgt gctgcaaggc gattaagttg ggtaacgcca gggttttccc agtcacgacg 540
   ttgtaaaacg acggccagtg ccaagcttat cgatttcgaa cccggggtac cgaatttcat 600
   tttgtttttt tctatgctat aaatagaatt cctcgaggtc gacggtatcg ataagcttga     660
   tatcgaattc ctgcagcccg ggggatccgc attccggtac tgttggtaaa atggaagacg     720
   ccaaaaacat aaagaaaggc ccggcgccat tctatcctct agaggatgga accgctggag     780
   agcaactgca taaggctatg aagagatacg ccctggttcc tggaacaatt gcttttacag     840
   atgcacatat cgaggtgaac atcacgtacg cggaatactt cgaaatgtcc gttcggttgg     900
   cagaagctat gaaacgatat gggctgaata caaatcacag aatcgtcgta tgcagtgaaa     960
   actctcttca attctttatg ccggtgttgg gcgcgttatt tatcggagtt gcagttgcgc    1020
   ccgcgaacga catttataat gaacgtgaat tgctcaacag tatgaacatt tcgcagccta    1080
   ccgtagtgtt tgtttccaaa aaggggttgc aaaaaatttt gaacgtgcaa aaaaaattac    1140
   caataatcca gaaaattatt atcatggatt ctaaaacgga ttaccaggga tttcagtcga    1200
   tgtacacgtt cgtcacatct catctacctc ccggttttaa tgaatacgat tttgtaccag    1260
   agtcctttga tcgtgacaaa acaattgcac tgataatgaa ttcctctgga tctactgggt    1320
   tacctaaggg tgtggccctt ccgcatagaa ctgcctgcgt cagattctcg catgccagag    1380
   atcctatttt tggcaatcaa atcattccgg atactgcgat tttaagtgtt gttccattcc    1440
   atcacggttt tggaatgttt actacactcg gatatttgat atgtggattt cgagtcgtct    1500
   taatgtatag atttgaagaa gagctgtttt tacgatccct tcaggattac aaaattcaaa    1560
   gtgcgttgct agtaccaacc ctattttcat tcttcgccaa aagcactctg attgacaaat    1620
   acgatttatc taatttacac gaaattgctt ctgggggcgc acctctttcg aaagaagtcg    1680
   gggaagcggt tgcaaaacgc ttccatcttc cagggatacg acaaggatat gggctcactg    1740
   agactacatc agctattctg attacacccg agggggatga taaaccgggc ggggtcggta    1800
   aagttgttcc attttttgaa gcgaaggttg tggatctgga taccgggaaa acgctgggcg    1860
   ttaatcagag aggcgaatta tgtgtcagag gacctatgat tatgtccggt tatgtaaaca    1920
   atccggaagc gaccaacgcc ttgattgaca aggatggatg gctacattct ggagacatag    1980
   cttactggga cgaagacgaa cacttcttca tagttgaccg cttgaagtct ttaattaaat    2040
   acaaaggata tcaggtggcc cccgctgaat tggaatcgat attgttacaa caccccaaca    2100
   tcttcgacgc gggcgtggca ggtcttcccg acgatgacgc cggtgaactt cccgccgccg    2160
   ttgttgtttt ggagcacgga aagacgatga cggaaaaaga gatcgtggat tacgtcgcca    2220
   gtcaagtaac aaccgcgaaa aagttgcgcg gaggagttgt gtttgtggac gaagtaccga    2280
   aaggtcttac cggaaaactc gacgcaagaa aaatcagaga gatcctcata aaggccaaga    2340
   agggcggaaa gtccaaattg taaaatgtaa ctgtattcag cgatgacgaa attcttagct    2400
   attgtaatcg gatccactag ttctagagcg gccgccaccg cggtggagct ccaattcgcc    2460
   ctatagtgag tcgtattgga atcccaggcg acggggcaaa tc                       2502
<210> 9
   <211> 826
   <212> DNA
   <213> Artificial
<220>
   <223> SFV DNA
<400> 9
   agaaacaggc atcttacgcg tgtcgattgt tgggaatctt gacaaaacat tccacgtttc     60
   catccgaaga atatttccca ctcgtgcgta gcattatgtc catgtacaac acgctgataa    120
   aagacgatat tatctggttt aaagagatca ccccgcatct gtacgagtat gtgatgtaca    180
   aacaaaacgt gaataacccc tgtttccaaa tttccaccgt agcggtaaat ctatcccgtc    240
   atgtacccaa atcttctaca gaacccacca agtatcaaac caagtctaaa caaatgaaaa    300
   cgagaatgat tacggacgtt gtatcgttcg acgaaaaatt gaagctcgat gaggcaatta    360
   tgtacaaaaa caaaaaggat tatttcgaaa ttaagaaact gtacatgagg ttgaagaaat    420
   ttgtacgtaa gaagaaatcg gtaaacgacg gtgtactctg tgacagagtt aaaatgattt    480
   acggacacat ccacgagata gaaagagtcg cagtcaatga atacgccatg actaagtcct    540
   tgttacatta cgtatttccg aatctgttta acgatgacaa gcatcacctg ttttacagat    600
   gcgacaaagt ggatggattg ggggtattat cgtctaagaa actcaatcta atcagagtca    660
   tattggaaaa cagattcaaa ataggcaaac aaaaatggac gatgttaaaa aagtacatcg    720
   acacggtgtg tagcacgggg aaaccgttat tgaagttggg aacgtatccg tattttaaac    780
   tgaaaccgtt gaacgccatc gtttccaatt accaaggcgt ttccgt                  826
<210> 10
   <211> 39
   <212> DNA
   <213> Artificial
<220>
   <223> pRP406 primer
<400> 10
   ggatctactg ggttaccaga aacaggcatc ttacgcgtg 39
<210> 11
   <211> 35
   <212> DNA
   <213> Artificial
<220>
   <223> pRP406 primer
<400> 11
   atcctttgta tttaattaaa cggaaacgcc ttggt 35